# EUROPEAN PATENT APPLICATION

(11) **EP 4 706 707 A1**
(43) Date of publication of application: **11.03.2026**
(21) Application number: 25199488.5
(22) Date of filing: 01.09.2025
(51) Int. Cl.: A61M 5/315, A61M 5/36, A61M 5/20, A61M 5/24

(54) **INJECTION DOSE ADJUSTMENT ASSEMBLY, INJECTION MECHANISM AND INJECTION SYRINGE**

(30) Priority: 06.09.2024 TW 113133901
(71) Applicant: CC Biotechnology Corporation, Tainan City, Taiwan 70955 (TW)
(72) Inventor: YEH, Chin-Min, Tainan City (TW); WANG, Chih-Wei, Tainan City (TW)
(74) Representative: Casalonga

(57) **Abstract**

An injection dose adjustment assembly (A) is connected with an injection assembly (B) to form an injection mechanism, and is further coupled with a vial mounting tube (D) to form an injection syringe. The injection dose adjustment assembly (A) has a mounting sleeve (20) mounted in an outer tube (10). Two resilient ratchet arms (21) of the mounting sleeve (20) abut against two different one-way ratchet teeth (121) of an inner ratchet portion (12) of the outer tube (10) respectively. An elastic transmission element (40) and a pressing element (60) are mounted on the mounting sleeve (20). A dose adjusting knob (50) is mounted on the outer tube (10) and is rotatable in a first direction and a second direction. By rotating the mounting sleeve (20) through the elastic transmission element (40), an injection dose can be adjusted and a moving distance of an injection push rod (70) of the injection assembly (B) can be controlled.

## Description

### 1. Field of the Invention

The present invention relates to an injection syringe for injecting a liquid medicament, especially to an injection dose adjustment assembly.

### 2. Description of the Prior Art(s)

In a structure of a conventional dose metering type syringe, a dose controlling structure of an injection is used for controlling dose of each injection and is locked after repeatable uses in predetermined times, thereby reminding users that the entire syringe must be discarded to ensure the safety of an injection pen.

However, the injection dose needs to be adjusted according to the needs of different injection objects. Lacking the function of adjusting the injection dose, the conventional dose metering syringe can hardly meet the needs of different users. To solve the problem, some of the conventional dose metering syringes have an injection mechanism that is capable of adjusting an injection dose, to meet the different injection dose requirements of different users.

However, an engaging structure based on a ratchet wheel and a pawl of an inner ratchet in the conventional syringe is an adjustment mechanism that is only able to drive an inner mounting tube to rotate in one direction (clockwise) through a dose adjusting knob and is unable to drive the inner mounting tube to rotate in a reverse direction (counterclockwise). Thus, when adjusting the injection dose, the user is only able to make incremental adjustment to a dose scale. Once a set increment exceeds a predetermined increment, it is impossible to rotate reversely to return to a predetermined dose scale, causing inconvenience to the user.

The main objective of the present invention is to provide an injection dose adjustment assembly, comprising an outer tube, a mounting sleeve, an elastic transmission element, a dose adjusting knob, and a pressing element.

The outer tube has an inner ratchet portion disposed on a rear end of the outer tube and multiple one-way ratchet teeth formed on an inner sidewall of the inner ratchet portion and extending radially. A notch is formed between each two of the one-way ratchet teeth that are disposed next to each other.

The mounting sleeve is mounted in the outer tube, is axially movable, and is rotatable in a first direction and a second direction opposite to the first direction. The mounting sleeve has two resilient ratchet arms being able to abut against two of the one-way ratchet teeth of the inner ratchet portion respectively.

The elastic transmission element is mounted on a rear end of the mounting sleeve and is able to provide an axial elastic preload.

The dose adjusting knob is rotatably mounted on the rear end of the outer tube and selectively engages with or disengages from the elastic transmission element.

The pressing element is mounted in the dose adjusting knob and is axially movable.

The main objective of the present invention is to provide an injection mechanism capable of adjusting an injection dose. The injection mechanism is configured to be connected to a medicine vial containing a liquid medicament, and comprises the injection dose adjustment assembly as described and an injection assembly. The injection assembly includes an injection push rod, a driving ring, a resilient element, a clutch control unit, and an injection syringe.

The injection push rod is mounted in the mounting sleeve of the injection dose adjustment assembly and is able to be driven to axially move forward to perform an action of injecting the liquid medicament in the medicine vial.

The driving ring is mounted in the mounting tube, is axially movable, and is spirally connected with the injection push rod. The mounting sleeve is able to change a position of the driving ring on the injection push rod.

The resilient element is mounted in the mounting sleeve and is disposed between the driving ring and an inner wall of the rear end of the mounting sleeve to provide a resilient force and to allow the mounting sleeve to inter-connect with the transmission ring. When the mounting sleeve is rotated, a resilient potential energy is formed in the resilient element that is driven by the driving ring.

The clutch control unit is mounted inside a front end of the outer tube. When the dose adjusting knob rotates the mounting sleeve, the clutch control unit limits a position of the injection push rod. When the mounting sleeve is triggered to be disconnected from the transmission ring, the clutch control unit is able to be connected with the mounting sleeve. When the injection push rod completes the action of injection, the resilient element drives the mounting sleeve to disconnect from the clutch control unit and be restored.

The main objective of the present invention is to provide an injection syringe capable of adjusting an injection dose. The injection syringe comprises the injection mechanism capable of adjusting the injection dose as described and a vial mounting tube sleeved on an outer side of the medicine vial and detachably mounted on the front end of the outer tube.

In the injection dose adjustment assembly, with the mounting sleeve mounted in the outer tube, the mounting sleeve is rotatable in the first direction and the second direction. Moreover, the elastic transmission element and the pressing element are mounted on the rear end of the mounting sleeve, and the dose adjusting knob is mounted on the rear end of the outer tube. The inner ratchet portion is formed on the rear end of the outer tube, the two resilient ratchet arms of the mounting sleeve abut against two different ones of the one-way ratchet teeth of the inner ratchet portion of the outer tube respectively, and the dose adjusting knob is rotatable in the first direction and the second direction. By rotating the mounting sleeve through the elastic transmission element, an injection dose can be adjusted and a moving distance of the injection push rod of the injection assembly can be controlled.

### IN THE DRAWINGS:

Fig. 1 is an exploded perspective view of an injection dose adjustment assembly in accordance with the present invention;
Fig. 2 is a cross-sectional side view of the injection dose adjustment assembly in Fig. 1, shown assembled;
Fig. 3 is a rear end view of an outer tube and a mounting sleeve of the injection dose adjustment assembly in Fig. 1, shown assembled;
Fig. 4 is an exploded perspective view of a transmission ring and a resilient transmission element of the injection dose adjustment assembly in Fig. 1;
Fig. 5A is a perspective view of the transmission ring of the injection dose adjustment assembly in Fig. 1;
Fig. 5B is another perspective view of the injection dose adjustment assembly in Fig. 1;
Fig. 6A is a perspective view of the resilient transmission element of the injection dose adjustment assembly in Fig. 1;
Fig. 6B is another perspective view of the resilient transmission element of the injection dose adjustment assembly in Fig. 1;
Fig. 7 is an enlarged and partial exploded perspective view of the injection dose adjustment assembly in Fig. 1, showing the transmission ring, the resilient transmission element, the mounting sleeve and the outer tube being assembled and an injection dose adjusting knob being separated;
Fig. 8 is an enlarged side view of the injection dose adjustment assembly in Fig. 1;
Fig. 9 is cross-sectional view of the injection dose adjustment assembly across line 9-9 in Fig. 8;
Fig. 10 is an exploded perspective view of an injection mechanism in accordance with the present invention;
Fig. 11 is a perspective view of the mounting sleeve and an injection assembly of the injection mechanism in Fig. 10, shown assembled;
Fig. 12 is a partial exploded perspective view of the mounting sleeve and the injection assembly in Fig. 11, showing an orientation control element and a piston abutting element being separated;
Fig. 13 is another partial exploded perspective view of the mounting sleeve and the injection assembly in Fig. 11, showing the orientation control element and the piston abutting element being separated;
Fig. 14 is an exploded view of the injection assembly and the mounting sleeve in Fig. 11;
Fig. 15 is another exploded view of the injection assembly and the mounting sleeve in Fig. 11;
Fig. 16 is a side view of the injection mechanism in Fig. 10;
Fig. 17 is a cross-sectional side view of the injection mechanism across line 17-17 in Fig. 16;
Fig. 18 is a cross-sectional end view of the injection mechanism across 18-18 in Fig. 16;
Fig. 19 is a cross-sectional end view of the injection mechanism across line 19-19 in Fig. 16;
Fig. 20 is a cross-sectional end view of the injection mechanism across line 20-20 in Fig. 16;
Fig. 21 is an exploded perspective view of an injection syringe in accordance with the present invention;
Fig. 22 is a perspective view of the injection syringe in Fig. 21, shown assembled;
Fig. 23 is a cross-sectional side view of the injection syringe in Figs. 21 and 22;
Fig. 24 is an operational reference scheme (1) of the injection syringe in Figs. 21 to 23;
Fig. 25 is an operational reference scheme (2) of the injection syringe in Figs. 21 to 23;
Fig. 26 is an operational reference scheme (3) of the injection syringe in Figs. 21 to 23;
Fig. 27 is an operational reference scheme (4) of the injection syringe in Figs. 21 to 23;
Fig. 28 is a cross-sectional side view of the injection syringe in Figs. 21 to 23, shown before performing injection;
Fig. 29 is an enlarged cross-sectional side view in partial section of the injection syringe in Fig. 28;
Fig. 30 is a cross-sectional side view of the injection syringe in Figs. 21 to 23, shown during injection;
Fig. 31 is an enlarged cross-sectional side view in partial section of the injection syringe in Fig. 30; and
Fig. 32 is a cross-sectional side view of the injection syringe in Figs. 21 to 23, showing liquid medicament in a medicine vial being used up.

According to the description of the summary of the invention, the present invention provides an injection dose adjustment assembly, an injection mechanism capable of adjusting an injection dose, and an injection syringe capable of adjusting an injection dose. Specific structures of the injection dose adjustment assembly, the injection mechanism and the injection syringe are described as follows.

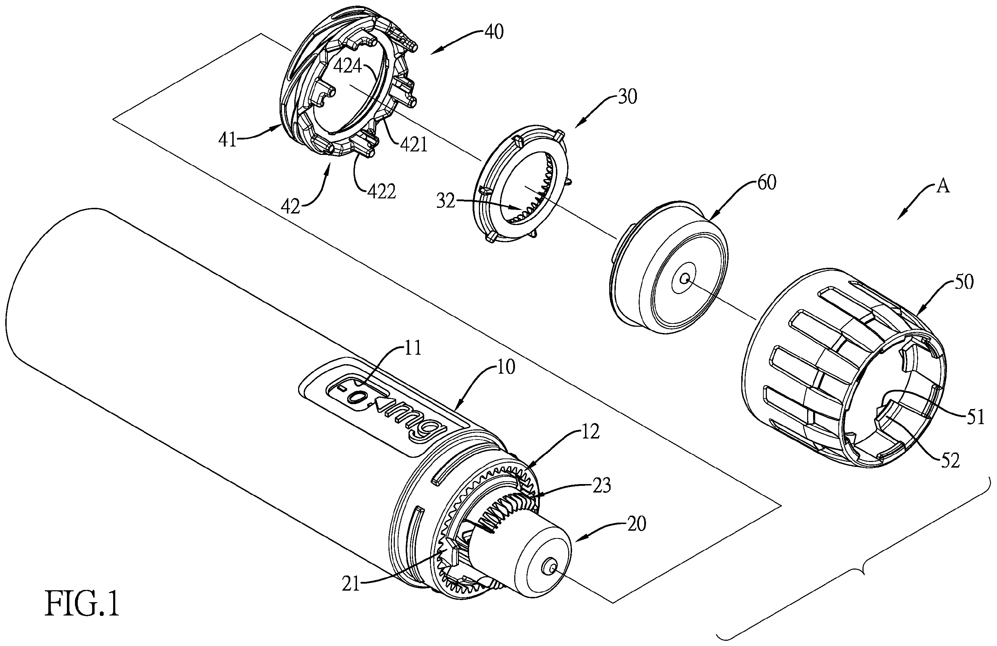

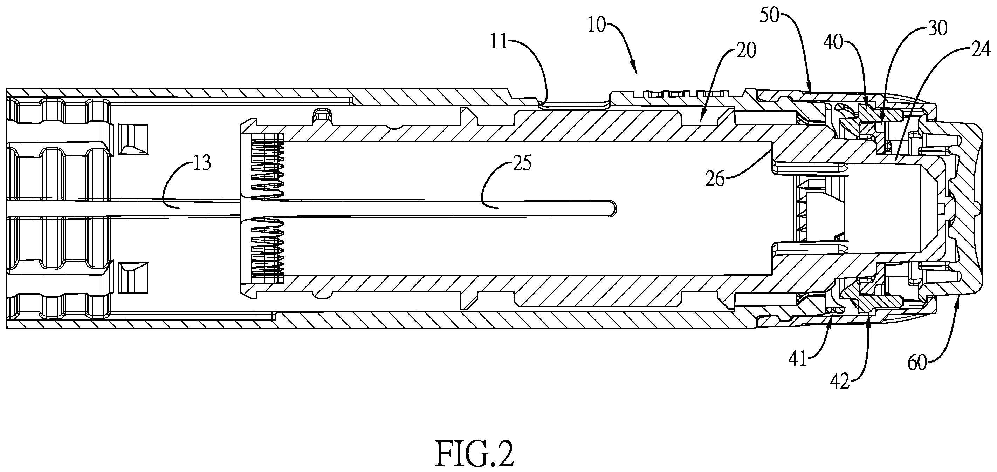

As shown in Figs. 1 and 2, an embodiment of the injection dose adjustment assembly A is disclosed. The injection dose adjustment assembly A comprises an outer tube 10, a mounting sleeve 20, an elastic transmission element 40, a dose adjusting knob 50 and a pressing element 60, or further comprises a transmission ring 30.

As shown in Figs. 1 to 3, the outer tube 10 has an inner ratchet portion 12 disposed on a rear end of the outer tube 10. Multiple one-way ratchet teeth 121 are formed on an inner sidewall of the inner ratchet portion 12 and extend radially.

As shown in Figs. 1 to 3, the mounting sleeve 20 is mounted in the outer tube 10, is axially movable, and is rotatable in a first direction and a second direction opposite to the first direction. When the first direction is clockwise, the second direction is counterclockwise, and vice versa. The mounting sleeve 20 has two resilient ratchet arms 21. The two ratchet arms 21 are able to abut against two of the one-way ratchet teeth 121 of the inner ratchet portion 12 respectively.

In the embodiment, an outer peripheral surface of the mounting sleeve 20 is further provided with multiple dosage scales of different values, such as "-0.- ", "0.10-", "0.20-", "0.25-", "0.50-", "0.75-," or an exhaust icon with "--- -" pattern between "-0.- (mg)" and "0.10-." The outer tube 10 has a scale window 11, and a text "mg" indicating dosage unit and a scale indicator. Any one of the dosage scales and the exhaust icon of the mounting sleeve 20 is able to be rotated to correspond in position to the scale window 11. Positions of each of the dosage scales representing a specific value and the exhaust icon are arranged on the mounting sleeve 20 according to a forward stroke of a corresponding injection dose.

As shown in Figs. 1 to 3, in the embodiment, in the inner ratchet portion 12 of the outer tube 10, a notch 120 is formed between each two of the one-way ratchet teeth 121 that are disposed next to each other. Each of the one-way ratchet teeth 121 has a forward tooth face 1211 facing toward the second direction and a backward tooth face 1212 facing toward the first direction. Each of the two resilient ratchet arms 21 has an engaging protrusion 210 formed on a distal end of the resilient ratchet arm 21. The engaging protrusion 210 has a first abutting surface 211 and a second abutting surface 212 inclined in different directions. The engaging protrusions 210 of the two resilient ratchet arms 21 are able to protrude in two of the notches 120 of the inner ratchet portion 12 of the outer tube 10 to occupy part of a space in said two engaging protrusions 210. The first abutting surface 211 of one of the resilient ratchet arms 21 abuts against the forward tooth face 1211 and the second abutting surface 212 of the other one of the resilient ratchet arms 21 abuts against the backward tooth face 1212. With the two resilient ratchet arms 21 abutting against two of the one-way ratchet teeth 121 of the inner ratchet portion 12, opposing forces are provided.

As described above, the forward tooth face 1211 and the backward tooth face 1212 of the one-way ratchet teeth 121 are inclined at different angles. Specifically, the backward tooth face 1212 is steeper than the forward tooth face 1211. The backward tooth face 1212 is capable of blocking the engaging protrusion 210 of the resilient ratchet arm 21 to move in a reverse direction.

In the embodiment, when the mounting sleeve 20 rotates in the first direction (clockwise), the engaging protrusions 210 of the two resilient ratchet arms 21 slide along the forward tooth faces 1211 of the one-way ratchet teeth 121 to move forward from one notch 120 to another. In the embodiment, when each of the two resilient ratchet arms 21 slides forward to the next notch 120, a first sound (e.g. a "beep" sound) is emitted. When the mounting sleeve 20 rotates in the second direction (counterclockwise), each of the two resilient ratchet arms 21 is blocked by the backward tooth face 1212 of a corresponding one of the one-way ratchet teeth 121. The two resilient ratchet arms 21 have to be switched to an unlocked state that weakens a reverse block effect first, and then the mounting sleeve 20 is rotated in the second direction to make the engaging protrusions 210 of the two resilient ratchet arms 21 slide along the backward tooth faces 1212 of the one-way ratchet teeth 121 to move backward from one notch 120 to another. In the embodiment, when the mounting sleeve 20 rotates in the second direction, since the reverse block effect of the two resilient ratchet arms 21 are weakened, a second sound (e.g. a "click" sound), which is different from the first sound, is emitted as each of the two resilient ratchet arms 21 slides backward to the next notch 120. Accordingly, when the injection dose adjustment assembly of the present invention is adjusted in the first direction and the second direction to increase or decrease the dose, recognizable and different snapping sounds are emitted, forming a sound-assisted rotation direction identification structure.

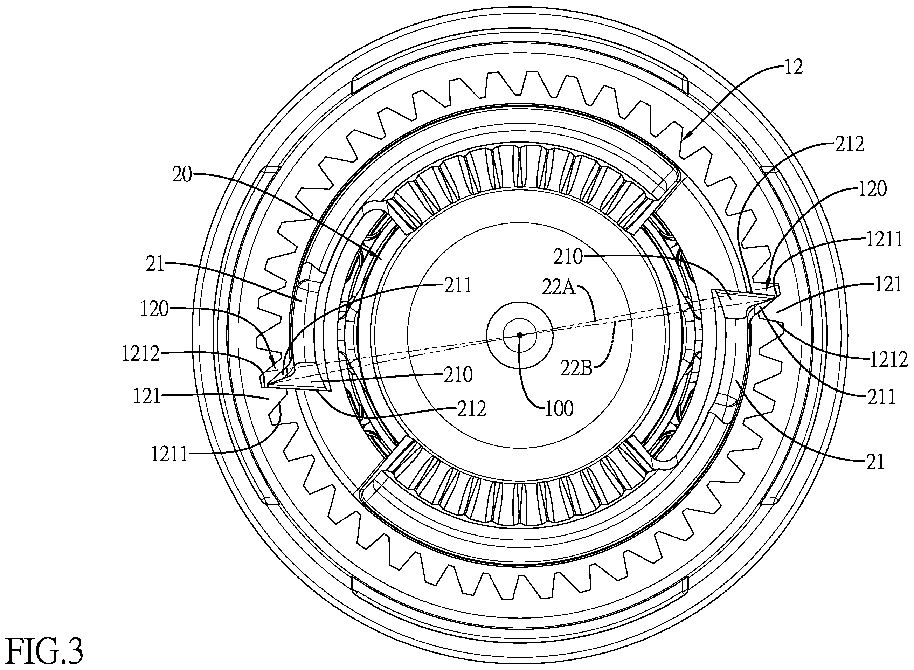

In order to make the mounting tube 20 reach the precision of "1U = 0.01ml" every time the mounting tube 20 steps one notch further, generally, a number of the one-way ratchet teeth 121 of the inner ratchet portion 12 is at least eighty-four. To prevent each of the one-way ratchet teeth 121 from being easily damaged due to its small size, as shown in Fig. 3, in the embodiment of the injection dose adjustment assembly A of the present invention, the inner ratchet portion 12 of the outer tube 10 has forty-two said one-way ratchet teeth 121 on a whole circumference and the two resilient ratchet arms 21 are arranged in a non-rotational symmetry. Specifically, the engaging protrusions 210 of the two resilient ratchet arms 21 protrude in two of the notches 120 of the inner ratchet portion 12 of the outer tube 10 respectively and each of the engaging protrusions 210 occupies half of a space of a corresponding one of the notches 120. A first connecting line 22A is defined to pass through one of the resilient ratchet arms 21 and a central axis 100 of the outer tube 10. A second connecting line 22B is defined to pass through the other one of the resilient ratchet arms 21 and the central axis 100 of the outer tube 10. An included angle defined between the first connecting line 22A and the second connecting line 22B is half of a central angle defined by the notch 120 with the central axis 100 of the outer tube 10 as a center. Thus, the inner ratchet portion 12 is equivalent to be divided into 84 sections, such that the size of each of the one-way ratchet teeth 121 is controlled and set within an appropriate size range and has appropriate mechanical strength, while meeting a requirement of "1U = 0.01ml" every time the mounting tube 20 steps one notch further.

As shown in Figs. 1, 2, 4 and 7, the elastic transmission element 40 is mounted on a rear end of the mounting sleeve 20 and is able to provide an axial elastic preload. The elastic transmission element 40 has a pushing portion 424. An axial force provided forward by the elastic transmission element 40 is able to be transferred to a radial pushing force applied on the engaging protrusions 210 of the two resilient ratchet arms 21 of the mounting sleeve 20 toward the central axis through the pushing portion 424. Accordingly, the two resilient ratchet arms 21 are pushed away from the one-way ratchet teeth 21 and are switched to the unlocked state with the weakened reverse block effect.

In the embodiment, the elastic transmission element 40 has an elastic portion 41 with compressive elasticity and an elasticity transmission annular portion 42 disposed on a rear end of the elastic portion 41. A front end of the elastic portion 41 abuts against the outer tube 10. The pushing portion 424 is disposed in the elasticity transmission annular portion 42. The elasticity transmission annular portion 42 is able to be forced to move forward and restore due to the elasticity of the elastic portion 41. The pushing portion 424 is formed as a conical surface that gradually tapers forward and is capable of pushing the engaging protrusions 210 of the two resilient ratchet arms 21 to move radially toward the central axis. When the dose adjusting knob 50 is released, the elasticity transmission annular portion 42 is restored due to the elasticity of the elastic portion 41, such that the two elastic ratchet arms 21 are also restored with their own elasticity and the engaging protrusions 210 engage in the inner ratchet portion 12 of the outer tube 10 again.

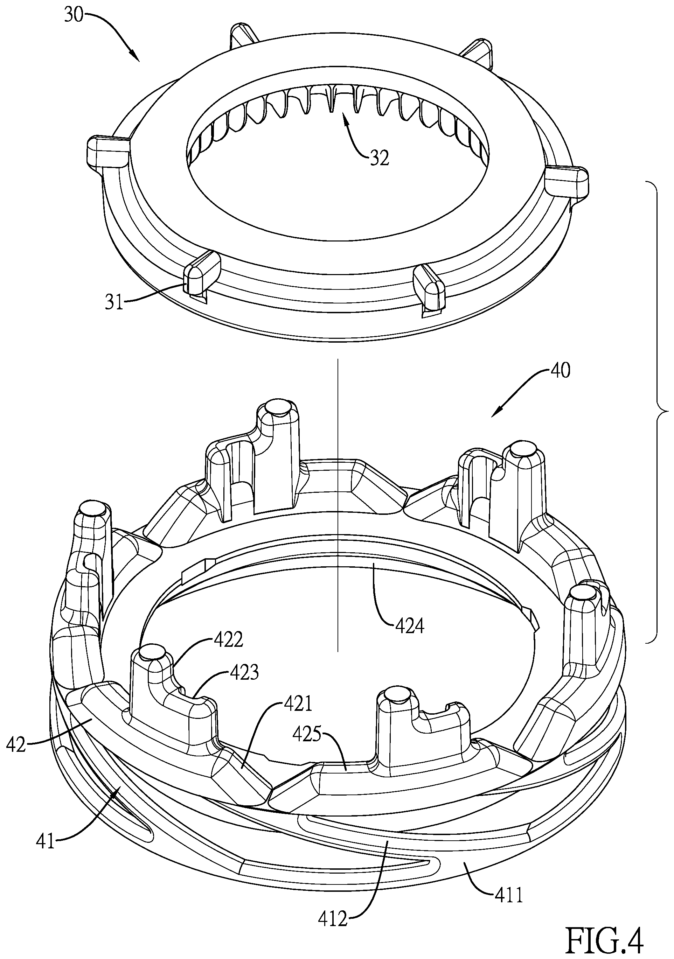

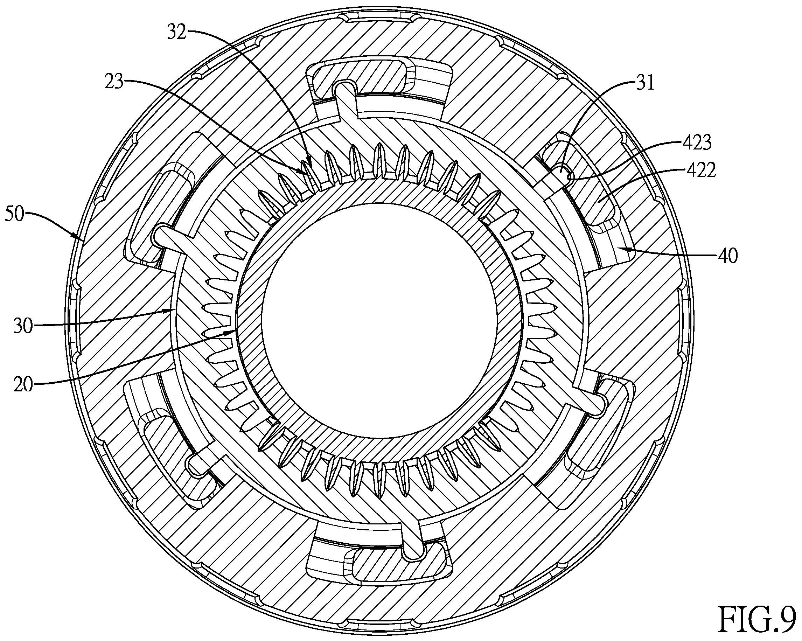

As shown in Figs. 4, 6A and 9, in the embodiment, the elastic portion 41 has an annular portion 411 and multiple elastic strips 412. The elastic strips 412 are rotationally symmetrically disposed between the elasticity transmission annular portion 42 and the annular portion 411. Each of the elastic strips 412 extends obliquely and is connected to the elasticity transmission annular portion 42 and the annular portion 411. The annular portion 411 abuts against the outer tube 10 and the elastic strips 412 provide the compressive elasticity.

As shown in Figs. 4, 6A and 9, in the embodiment, multiple dividing columns 422 are disposed on an outer peripheral edge of the elasticity transmission annular portion 42 of the elastic transmission element 40 and protrude backward. The elastic transmission element 40 is driven to move through the dividing columns 422.

As shown in Figs. 1, 2 and 7, the dose adjusting knob 50 is mounted on the rear end of the outer tube 10 and is rotatable at a fixed point. The dose adjusting knob 50 is capable of being turned in the first direction and the second direction, and is able to drive the mounting sleeve 20 to rotate in the same direction through the elastic transmission element 40 or is able to drive the mounting sleeve 20 to rotate in the same direction through the elastic transmission element 40 in combination with the transmission ring 30, so as to adjust the dosage scale with a predetermined value to correspond in position to the scale window 11 and to control by increasing or decreasing the injection dose of liquid medicament.

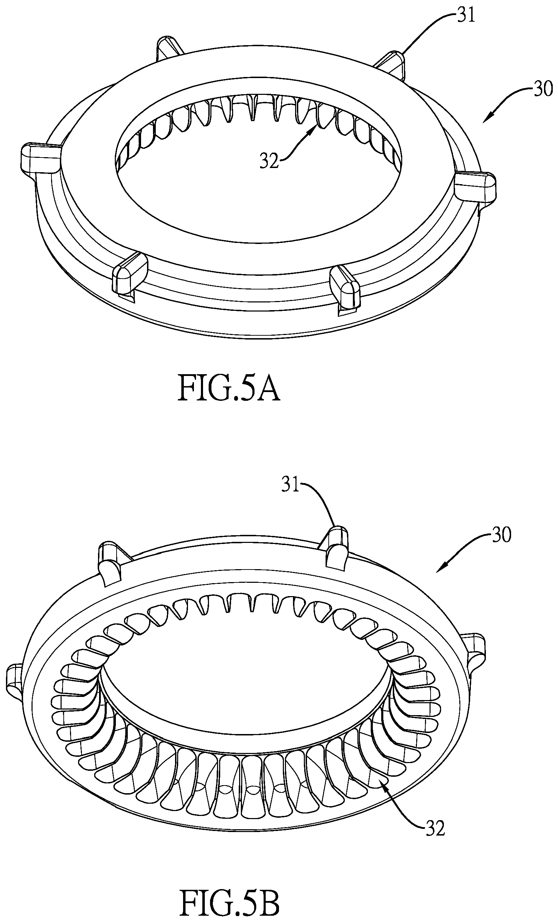

When the dose adjusting knob 50 drives the mounting sleeve 20 to rotate in the same direction through the elastic transmission element 40 in combination with the transmission ring 30, as shown in Figs. 1, 2 and 4 to 7, the transmission ring 30 is mounted on the rear end of the mounting sleeve 20 and selectively engages with or disengages from the mounting sleeve 20 along an axial direction. When the dose adjusting knob 50 is turned, the mounting sleeve 20 is driven to rotate through the elastic transmission element 40 and the transmission ring 30, and the mounting sleeve 20 is able to be triggered to disengage from the transmission ring 30.

As described above, the elasticity transmission annular portion 42 of the elastic transmission element 40 is connected to the transmission ring 30, the elastic transmission element 40 is located between the transmission ring 30 and the outer tube 10 to provide the axial elastic preload, and the elastic transmission element 40 and the transmission ring 30 are coupled together and rotate simultaneously. The transmission ring 30 also selectively engages with or disengages from the mounting sleeve 20 along the axial direction. In the embodiment, an inner annular engagement portion 32 is formed on an inner peripheral surface of the transmission element 30 and an engaging toothed portion 23 is formed on the rear end of the mounting sleeve 20. The engaging toothed portion 23 selectively engages with or disengages from the inner annular engagement portion 32.

In the embodiment, an outer peripheral edge of the transmission ring 30 is connected with the dividing columns 422 of the elasticity transmission annular portion 42. Each of the dividing columns 422 of the elasticity transmission annular portion 42 has a connecting groove 423 extending axially. The outer peripheral edge of the transmission ring 30 is provided with multiple connecting tabs 31. Each of the connecting tabs 31 corresponds in position to and is assembled in a corresponding one of the connecting grooves 423, so as to allow the transmission ring 30 to be connected with the elastic transmission element 40.

As shown in Figs. 1, 2 and 7, multiple pushing protrusions 51 are disposed on an inner peripheral sidewall of the dose adjusting knob 50. The elasticity transmission annular portion 42 has multiple coupling recesses 421 separately arranged and multiple annular backs 425. Each of the annular backs 425 is disposed on a side, which faces toward the second direction, of a corresponding one of the coupling recesses 421. Each of the pushing protrusions 51 corresponds in position to and selectively engages with or disengages from a corresponding one of the coupling recesses 421. Moreover, through mutual push between the pushing protrusions 51 and the coupling recesses 421, a forward axial force is provided to the elasticity transmission annular portion 42 of the elastic transmission element 40, so as to compress the elastic portion 41 and to store elastic potential energy.

As shown in Figs. 1, 7 and 10, multiple driving portions 52 are further provided on the inner peripheral sidewall of the dose adjusting knob 50. When the dose adjusting knob 50 is rotated in the first direction and the second direction, the pushing portions 52 abut against the dividing columns 422 of the elastic transmission element 40 to drive the elastic transmission element 40 to rotate.

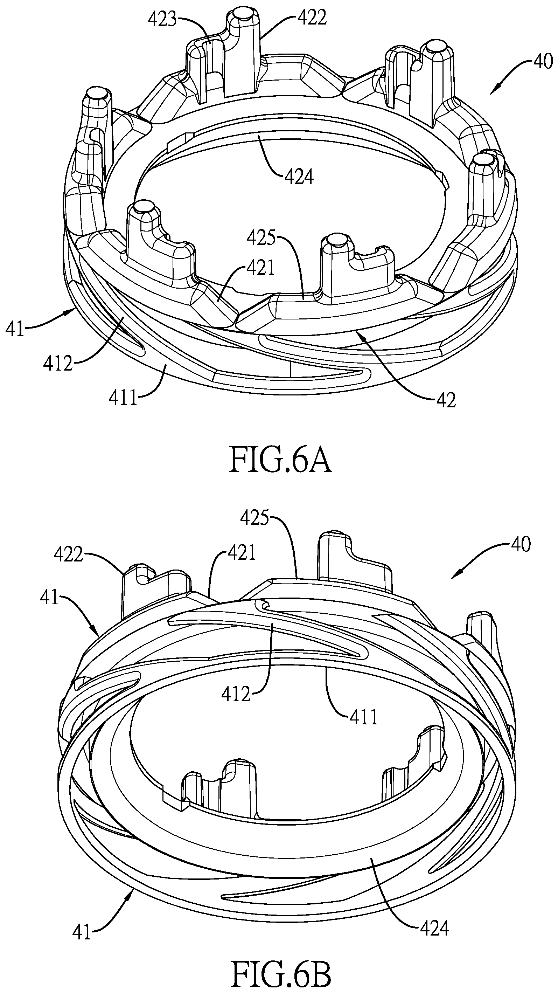

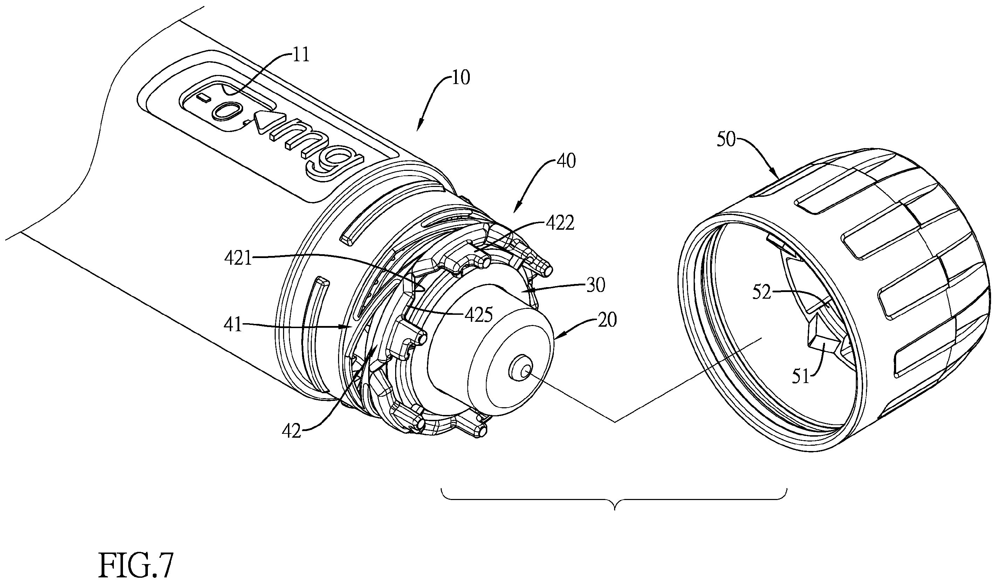

As shown in Figs. 6A, 6B and 7, in the embodiment, each of the pushing protrusions 51 on the inner peripheral sidewall of the dose adjusting knob 50 is formed as a triangular block with two inclined surfaces on two sides. Each of the coupling recesses 421 has a V-shaped bottom and corresponds in shape to the corresponding one of the pushing protrusions 51. On the elasticity transmission annular portion 42, one of the coupling recesses 421 and one of the annular backs 425 are disposed between two of the dividing columns 422 that are disposed next to each other. Said annular back 425 is disposed on a side, which faces toward the second direction, of said coupling recess 421. The driving portions 52 of the dose adjusting knob 50 are positioned behind the pushing protrusions 51. A width of each of the driving portions 52 is smaller than a distance defined between two of the dividing columns 422 of the elastic transmission element 40 that are disposed next to each other.

Thus, when rotated in the first direction, the dose adjusting knob 50 directly pushes the dividing columns 422 of the elastic transmission element 40 with the driving portions 52 to drive the elastic transmission element 40 to rotate. When the dose adjusting knob 50 is rotated in the second direction, with the mutual push between the triangular pushing protrusions 51 and the V-shaped bottoms of the coupling recesses 421 of the elasticity transmission annular portion 42, the axial force provided on the elasticity transmission annular portion 42 is formed to compress the elastic portion 41 and to store the elastic potential energy. Afterwards, the driving portions 52 of the dose adjusting knob 50 push the dividing columns 422 of the elastic transmission element 40 to drive the elastic transmission element 40 to rotate.

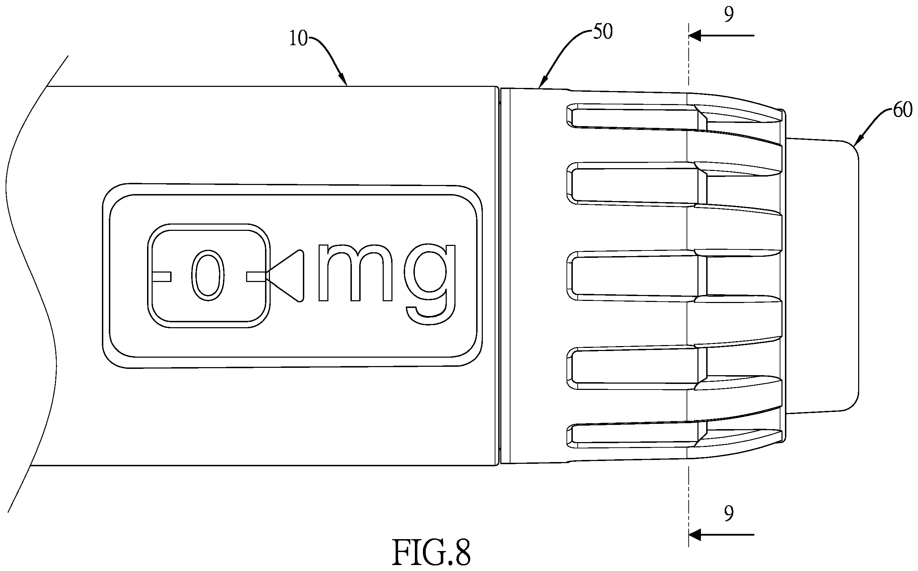

As shown in Figs. 1, 2 and 8, the pressing element 60 is mounted in the dose adjusting knob 50 and is axially movable. The pressing element 60 is able to trigger the mounting sleeve 20 to release the mounting sleeve 20 from directly or indirectly connecting with the dose adjusting knob 50, so as to perform an action of injection. In the embodiment, the transmission ring 30 is mounted on the rear end of the mounting sleeve 20 and selectively engages with or disengages from the mounting sleeve 20. When rotated, the dose adjusting knob 50 drives the mounting sleeve 20 to rotate through the elastic transmission element 40 and the transmission ring 30. The pressing element 60 is able to trigger the mounting sleeve 20 to disconnect from the transmission ring 30, making the mounting tube 20 disconnect from the dose adjusting knob 50 and not to be driven.

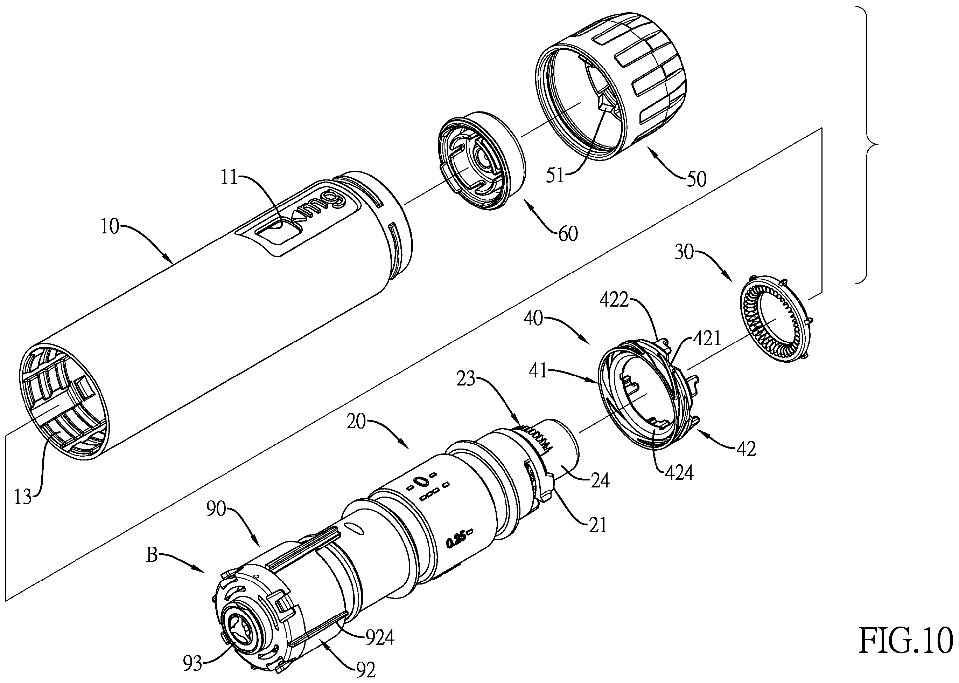

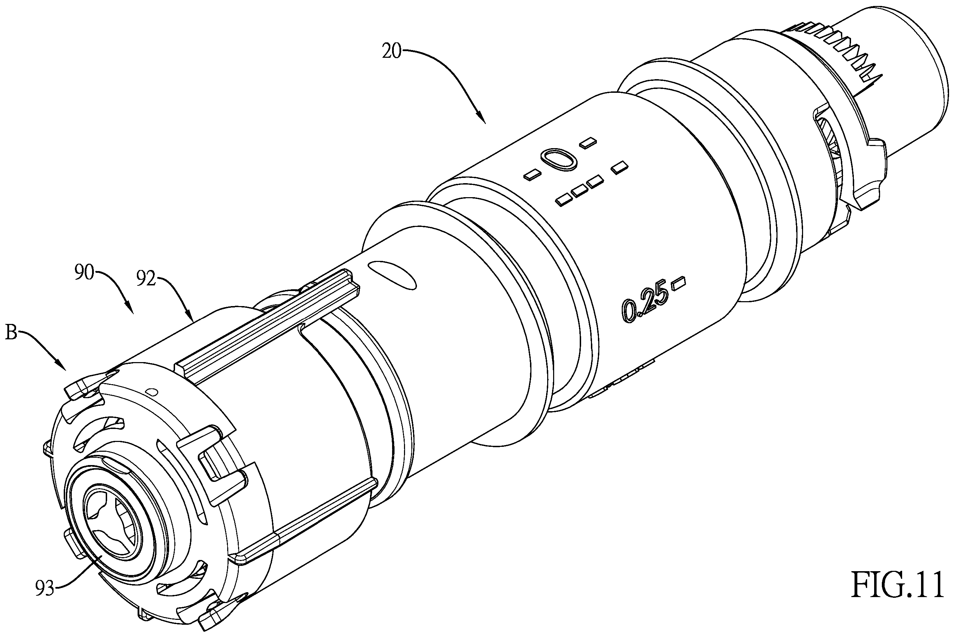

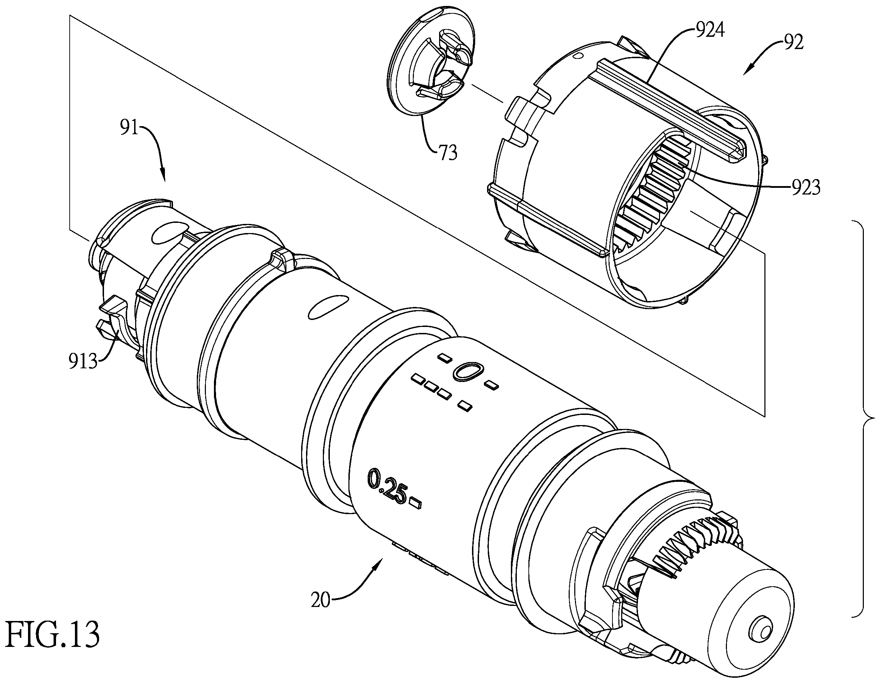

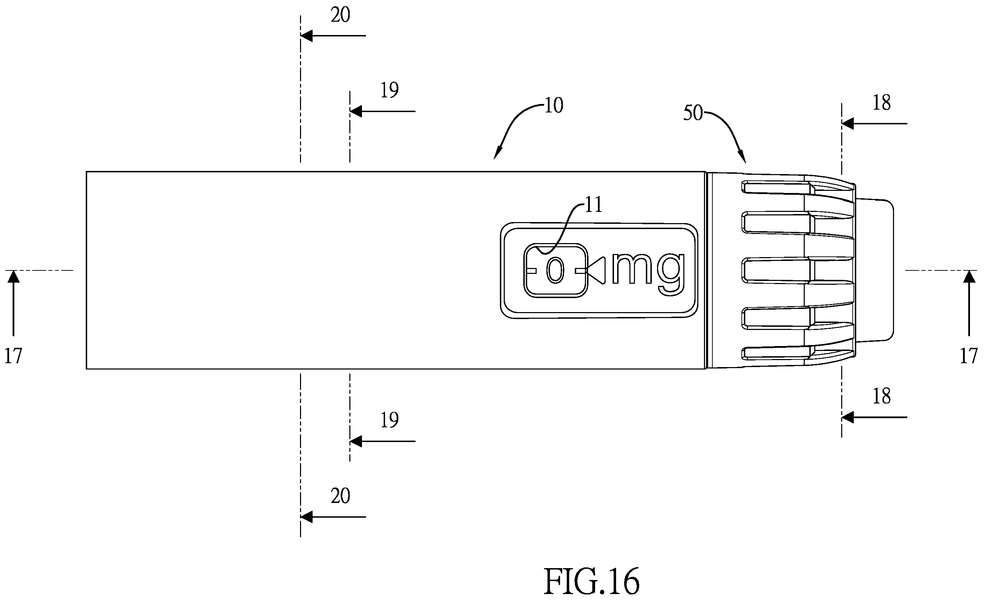

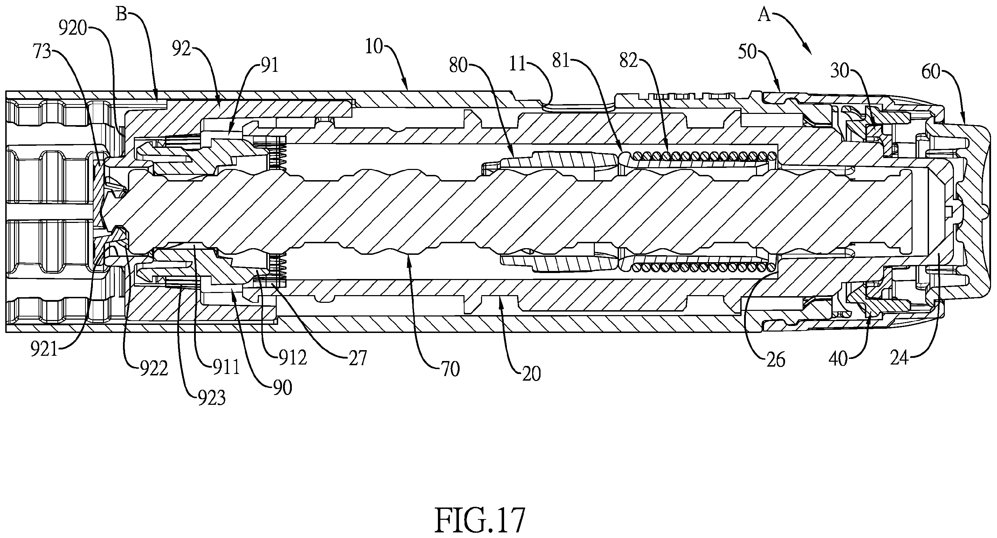

As shown in Figs. 10 to 17, an embodiment of the injection mechanism capable of adjusting an injection dose in accordance with the present invention is disclosed. The injection mechanism is able to be connected to a medicine vial C containing a liquid medicament and comprises the injection dose adjustment assembly A and an injection assembly B. A structure of the injection dose adjustment assembly A is as described above and will not be repeated here. The injection assembly B includes an injection push rod 70, a driving ring 80, a resilient element 82 and a clutch control unit 90.

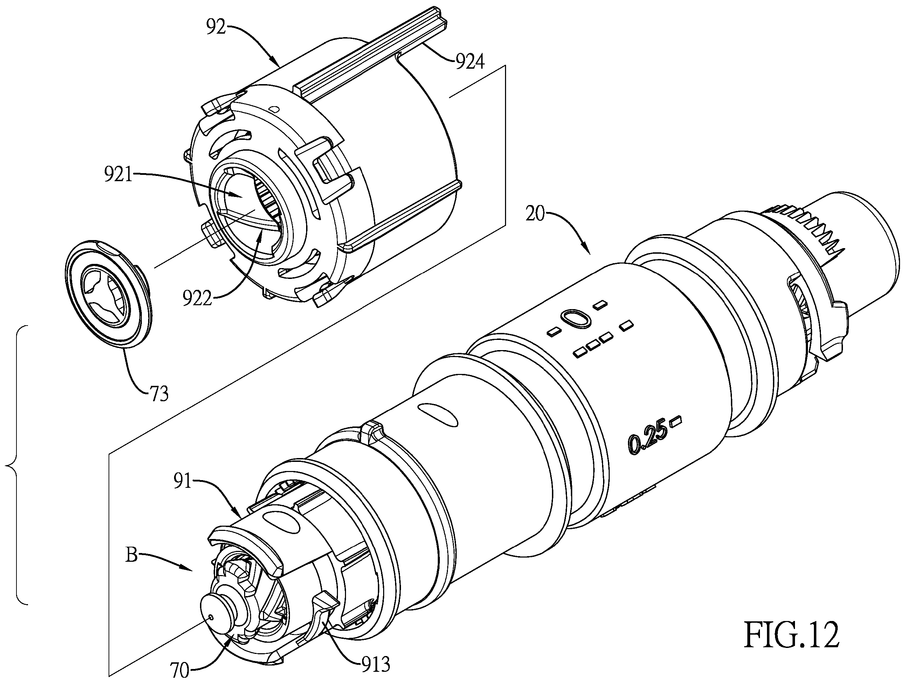

As shown in Figs. 12 to 17, the injection push rod 70 is mounted in the mounting sleeve 20 of the injection dose adjustment assembly A and is able to be driven to rotate and axially move forward to perform an action of injecting the liquid medicament in the medicine vial C. In the embodiment, an outer peripheral surface of the injection push rod 70 is provided with a first spiral guiding groove 71 and a second spiral guiding groove 72 that spiral in different directions. A spiraling direction of the first spiral guiding groove 71 corresponds to the first direction of the mounting sleeve 20. A spiraling direction of the second spiral guiding groove 72 corresponds to the second direction of the mounting sleeve 20. A piston abutting element 73 may be mounted on a front end of the injection push rod 70 and is configured to push a piston in the medicine vial C.

As shown in Figs. 12 to 17, the driving ring 80 is mounted in the mounting tube 20, is axially movable, and is connected with the injection push rod 70 through the first and second spiral guiding grooves 71, 72. The mounting sleeve 20 is able to change a position of the driving ring 80 on the injection push rod 70. In the embodiment, the driving ring 80 has a first spiral portion 801. The first spiral portion 801 is formed in the driving ring 80 and is coupled with the first spiral guiding groove 71. In addition, at least one axial guiding portion 802 is formed on an outer side surface of the driving ring 80 and protrudes in at least one movable guiding groove 25 of the mounting sleeve 20. Thus, the driving ring 80 is rotatable along with the mounting sleeve 20 and is movable forward and backward axially.

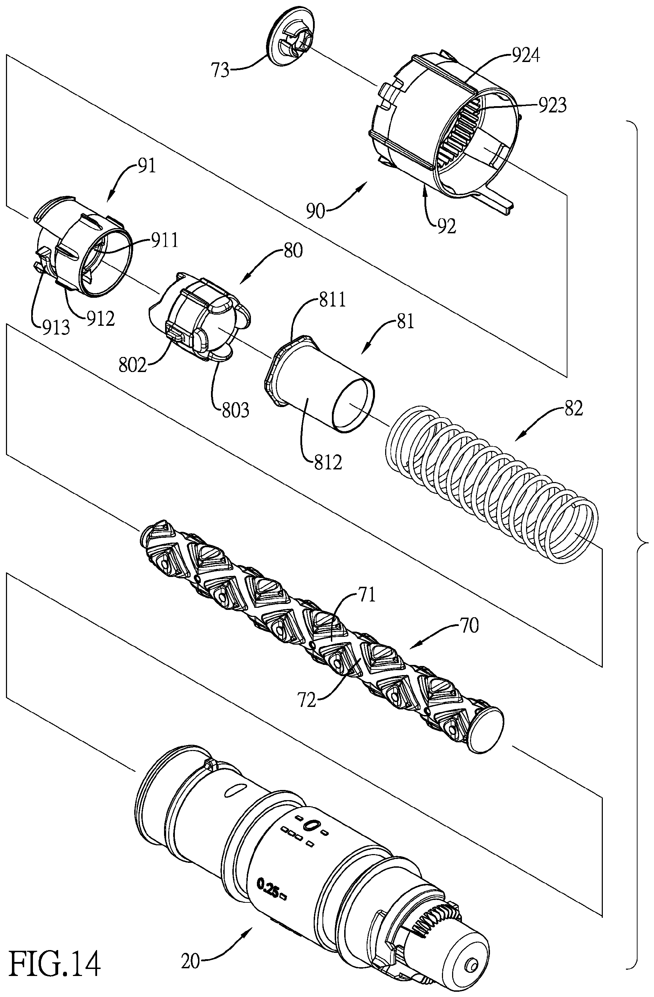

As shown in Figs. 14 to 17, the resilient element 82 is mounted in the mounting sleeve 20 and is disposed between the driving ring 80 and an inner wall of the rear end of the mounting sleeve 20 to provide a resilient force. In the embodiment, the resilient element 82 is a compression spring and an abutting tube 81 is mounted on a front end of the resilient element 82. The abutting tube 81 is sleeved on an outer side of the injection push rod 70. The front end of the resilient element 82 is connected to a rear end of the driving ring 80 through the abutting tube 81. In addition, an abutting sidewall 26 is provided on the inner wall of the rear end of the mounting sleeve 20. A rear end of the resilient element 82 abuts against the abutting sidewall 26 in the mounting sleeve 20. The resilient element 82 is disposed between the driving ring 80 and the inner wall of the rear end of the mounting sleeve 20 to provide the resilient force, to allow the mounting sleeve 20 to inter-connect with the transmission ring 30. When the mounting sleeve 20 is rotated, a resilient potential energy is formed in the resilient element 82 that is driven by the driving ring 80.

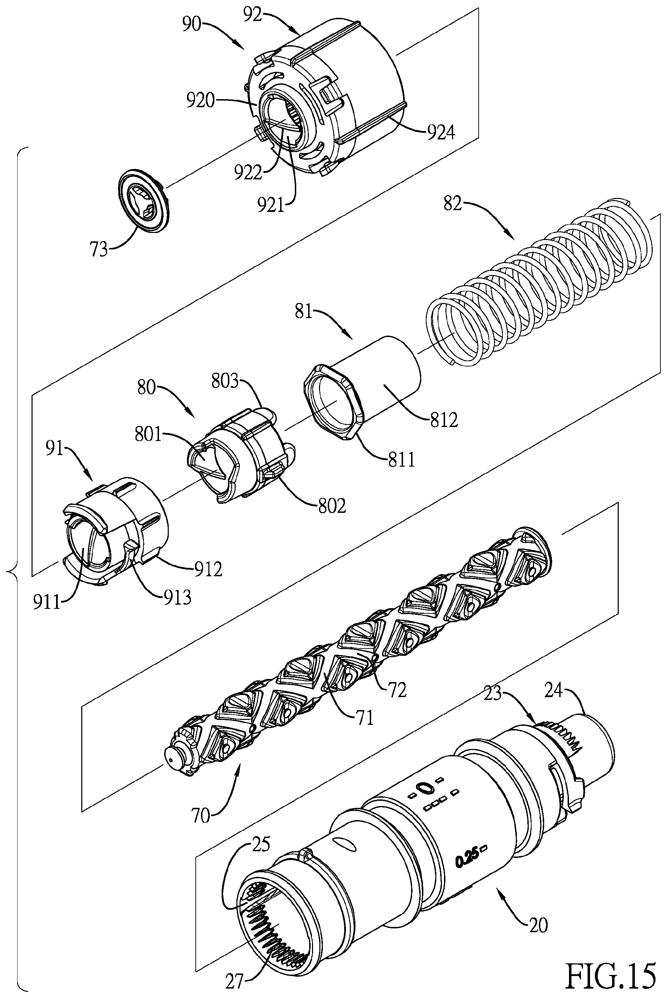

As shown in Figs. 14 and 15, multiple abutting protrusions 803 may be formed on and arranged around the rear end of the driving ring 80. The abutting protrusions 803 abut on a front end of the abutting tube 81. A distal end of each of the abutting protrusions 803 is formed as a concave curved surface, so as to reduce a contact area between the driving ring 80 and the abutting tube 81.

As shown in Figs. 10 to 17, the clutch control unit 90 is mounted inside a front end of the outer tube 10. When the dose adjusting knob 50 rotates the mounting sleeve 20, the clutch control unit 90 limits a position of the injection push rod 70. When the mounting sleeve 20 is triggered to be disconnected from the transmission ring 30, the clutch control unit 90 is able to be connected with the mounting sleeve 20. When the injection push rod 70 completes the action of injection, the resilient element 82 drives the mounting sleeve 20 to disconnect from the clutch control unit 90 and be restored.

As shown in Figs. 10 to 17, the clutch control unit 90 includes a clutch control ring 91 and an orientation control element 92. The clutch control ring 91 is mounted in front of the mounting sleeve 20 and the transmission ring 30. A second spiral portion 911 is formed in the clutch control ring 91. The second spiral portion 911 and the first spiral portion 801 of the driving ring 80 spiral in different directions. The injection push rod 70 is mounted in the clutch control ring 91 and is connected with the clutch control ring 91 with the second spiral portion 911 and the second spiral guiding groove 72 engaging with each other. The clutch control ring 91 is operable to selectively engage with or disengage from the mounting sleeve 20.

In the embodiment shown in Figs. 14, 15 and 19, multiple aligning teeth 912 and multiple resilient engaging portions 913 are formed on an outer peripheral surface of the clutch control ring 91. Multiple aligning notches 27 are formed in an inner peripheral surface of the front end of the mounting sleeve 20. When the mounting sleeve 20 is driven to move forward, the aligning teeth 912 of the clutch control ring 91 engage in the aligning notches 27 inside the front end of the mounting sleeve 20. When the mounting sleeve 20 is driven to move backward, the aligning teeth 912 of the clutch control ring 91 disengage from the aligning notches 27 inside the front end of the mounting sleeve 20.

As shown in Figs. 14 and 15, the orientation control element 92 is mounted inside the front end of the outer tube 10 and limits the clutch control ring 10 to be driven to rotate in the second direction only at a fixed position. The orientation control element 92 has a middle hole 921 and a first spiral engaging portion 922 disposed in the middle hole 921. The injection push rod 70 is mounted in the orientation control element 92 with the first spiral engaging portion 922 coupled with the first spiral guiding groove 71.

As shown in Figs. 14, 15 and 19, an inner peripheral surface of the orientation control element 92 is provided with multiple single bevel engaging teeth 923 arranged annularly. A front end of the clutch control ring 91 is pivotally connected to the orientation control element 92. The resilient engaging portions 913 of the clutch control ring 91 engage with the single bevel engaging teeth 923 respectively to limit the clutch control ring 91 to limit the clutch control ring 10 to be driven to rotate in the second direction only at a fixed position.

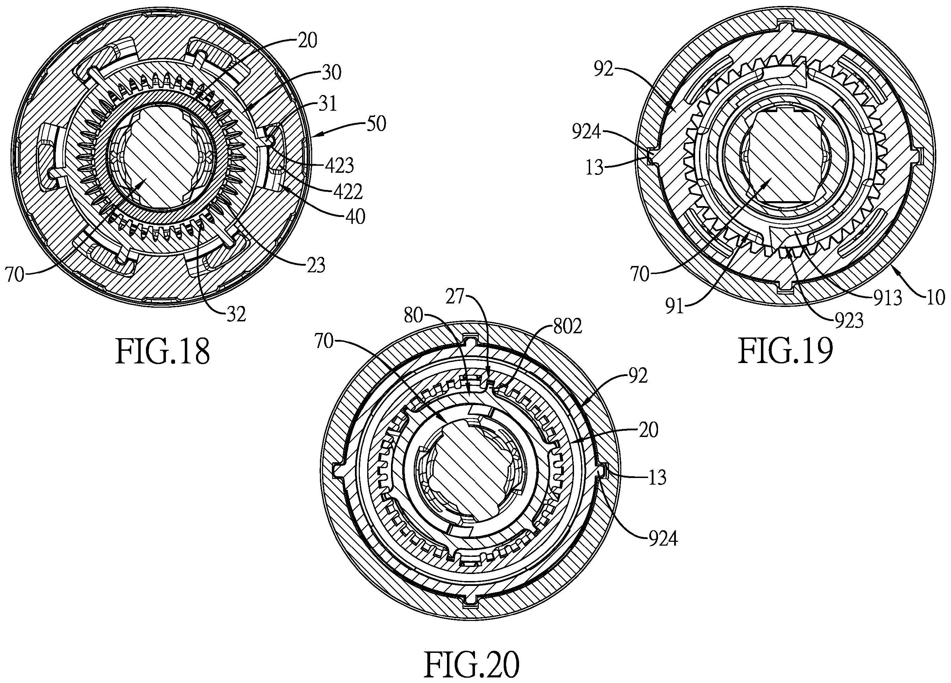

As shown in Figs. 10 and 20, at least one axial recess 13 is formed on a front section of an inner peripheral surface of the outer tube 10. At least one orientation rib 924 is formed on an outer peripheral surface of the orientation control element 92 and is fitted with the at least one axial recess 13. Thus, the orientation control element 92 is assembled in position in the front end of the outer tube 10 and is non-rotatable.

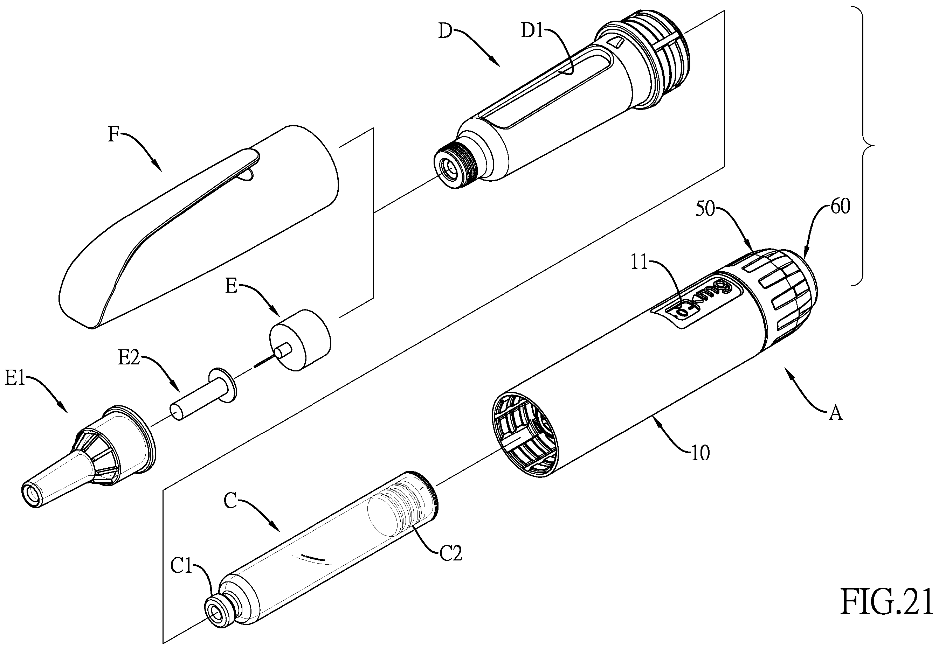

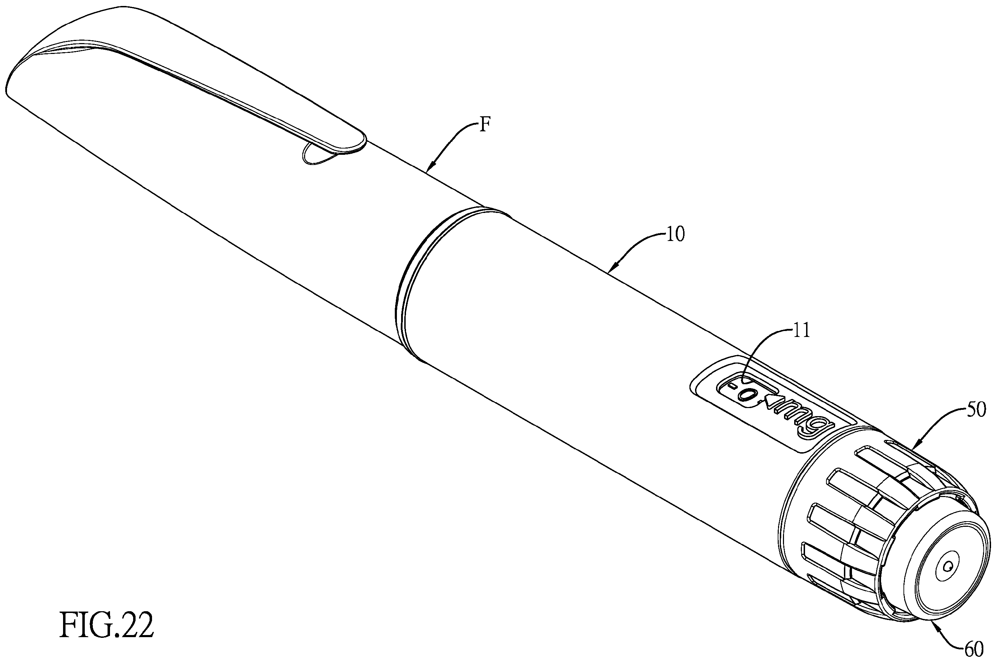

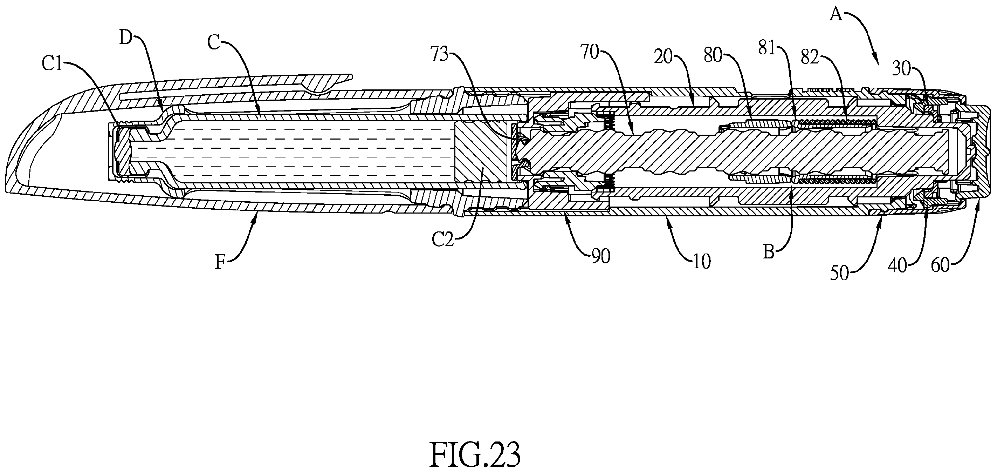

As shown in Figs. 21 to 23, an embodiment of the injection syringe capable of adjusting an injection dose in accordance with the present invention is disclosed. The injection syringe comprises the injection mechanism and a vial mounting tube D. A specific structure of the injection mechanism is as described above and will not be repeated here. The vial mounting tube D is sleeved on an outer side of the medicine vial C and is detachably mounted on the front end of the outer tube 10. A needle E is mounted on a front end of the vial mounting tube D and is able to pierce into the medicine vial C through the front end of the vial mounting tube D. At least one vial window D1 may be formed in a peripheral sidewall of the vial mounting tube D and is configured for a user to view status of liquid medicament in the medicine vial C directly through the at least one vial window D1.

As shown in Fig. 21, the at least one vial window D1 includes one vial window D1 formed in the vial mounting tube D for viewing the medicine vial C. The needle E is mounted on the front end of the vial mounting tube D and pierces into the medicine vial C. A needle cap E2 is mounted on and outside the needle E, and a needle protective sleeve E1 is sleeved on the needle cap E2 that covers the needle E. A protective cap F may be mounted on and outside the vial mounting tube D to provide protection for the vial mounting tube D and the medicine vial C inside the vial mounting tube D. When the injection syringe is formed as a pen, the protective cap F is formed as a pen cap. When the vial mounting tube D is disposed in the protective cap F, the user places the medicine vial C in the vial mounting tube D inside the protective cap F and then holds the protective cap F with his/her hand to insert a rear end of the vial mounting tube D into the front end of the outer tube 10.

For conciseness in explaining operation and usage of the injection dose adjustment assembly A, the injection mechanism and the injection syringe of the present invention, the entire injection syringe is used as a main body in the following explanation. The first direction (clockwise) is defined to increase the injection dose and the second direction (counterclockwise) is defined to decrease the injection dose. Further explanation is as follows.

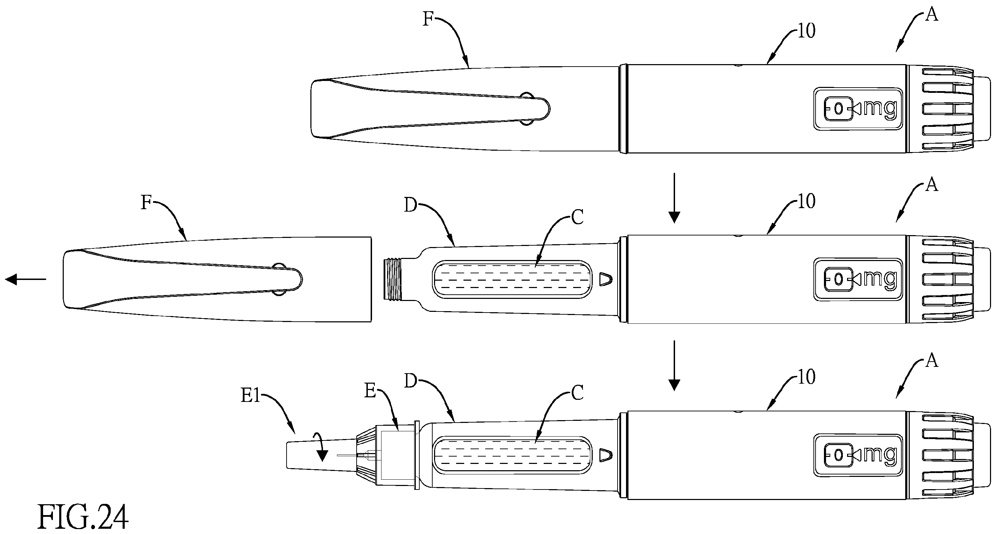

As shown in Fig. 24, the injection syringe is in an initial state. The medicine vial C is attached to a front end of the injection mechanism through the vial mounting tube D. The protective cap F is mounted on and outside the vial mounting tube D containing the medicine vial C. The dosage scale "0.- (mg)" corresponds in position to the scale window 11 of the outer tube 10. The protective cap F has to be removed before use, the needle protective sleeve E1 with the needle E and the needle cap E2 mounted therein is mounted to the front end of the vial mounting tube D, and the needle E is mounted on the front end of the vial mounting tube D and pierces into the medicine vial C.

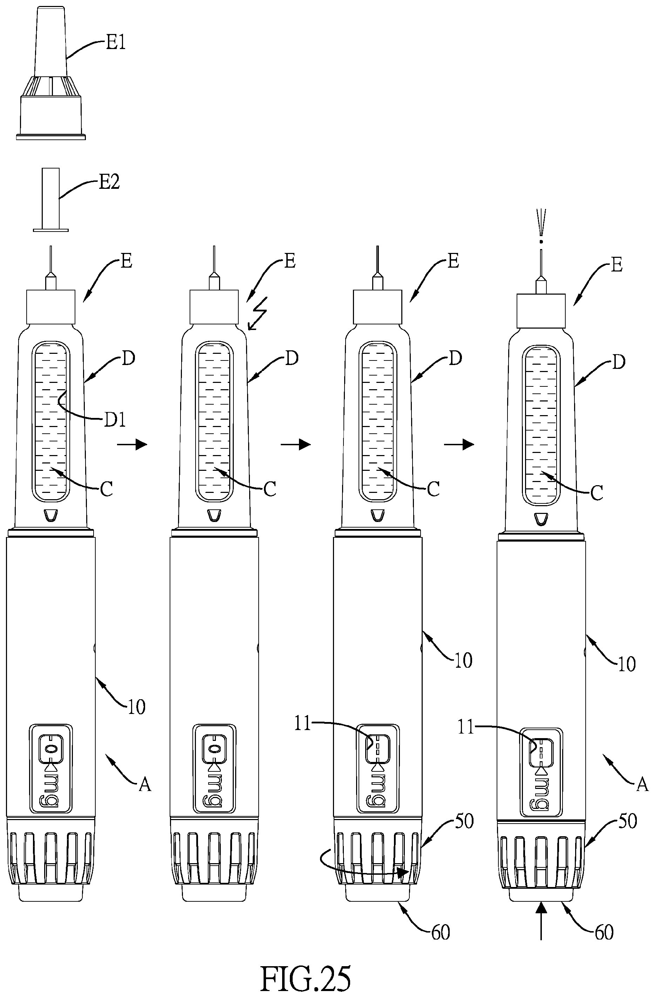

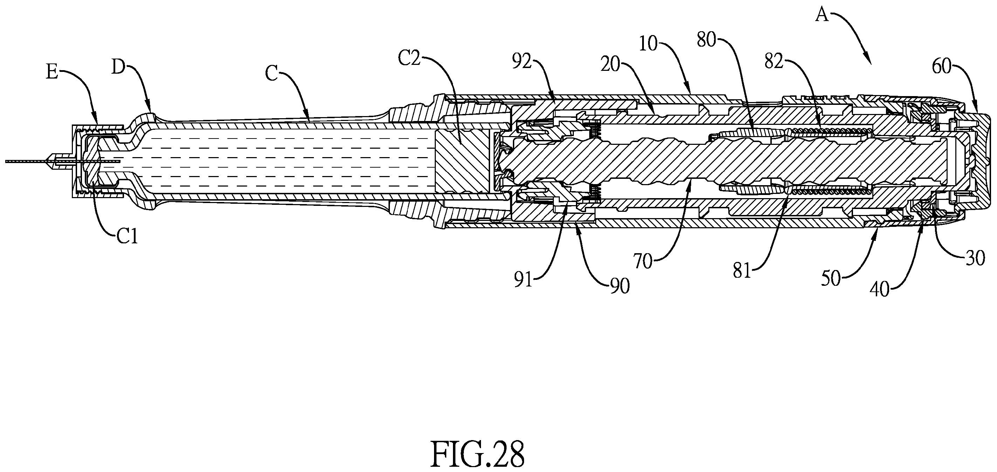

As shown in Figs. 25 to 28 and 31, in order to ensure that air in bubbles remaining in the medicine vial C would not be injected into the human body, before injection, a front end of the medicine vial C with the needle E is pointed upwards, the needle protective sleeve E1 and the needle cap E2 mounted outside the needle E are removed, and the vial mounting tube D mounted outside the medicine vial C is flicked by a finger several times. Thus, the bubbles inside the medicine vial C move upward to the front end of the medicine vial C. Afterwards, by turning the dose adjusting knob 50 to drive the dose scale of the mounting sleeve 20 to rotate from "0.-(mg)" to the exhaust icon "--- -" and correspond in position to the scale window 11 of the outer tube 10. With the front end of the medicine vial C with the needle E pointed upwards, by pressing the pressing element 60 on a rear end of the injection syringe, the injection push rod 70 in the injection mechanism is driven to push the piston in the medicine vial C. Accordingly, the bubbles in the medicine vial C can be completely expelled.

As described above, the processes of making the dose adjusting knob 50 drive the exhaust icon "--- -" of the mounting sleeve 20 to the scale window 11 of the outer tube and pressing the pressing element 60 to drive the injection push rod 70 to expel the bubbles in the medicine vial C are substantially the same as the action of injection. Therefore, a further description about said processes is omitted.

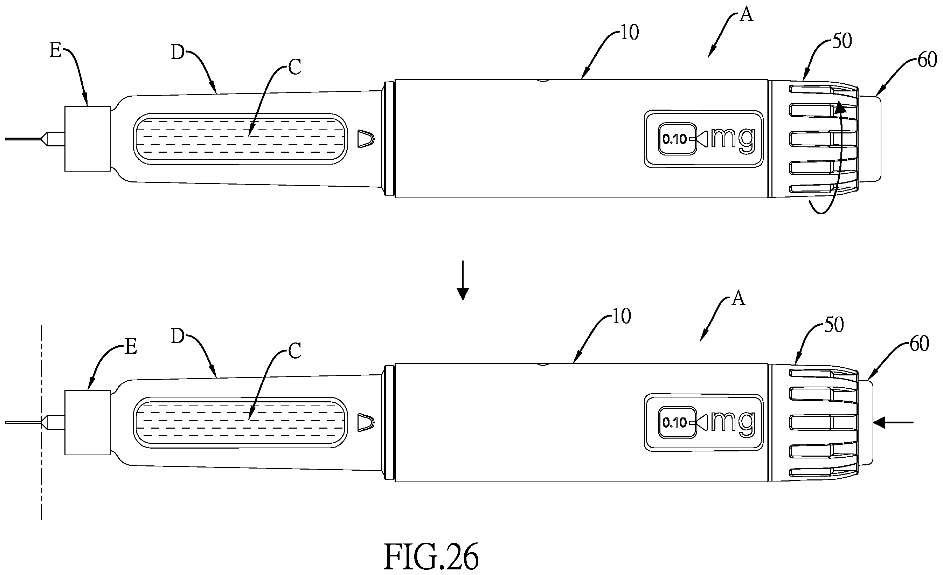

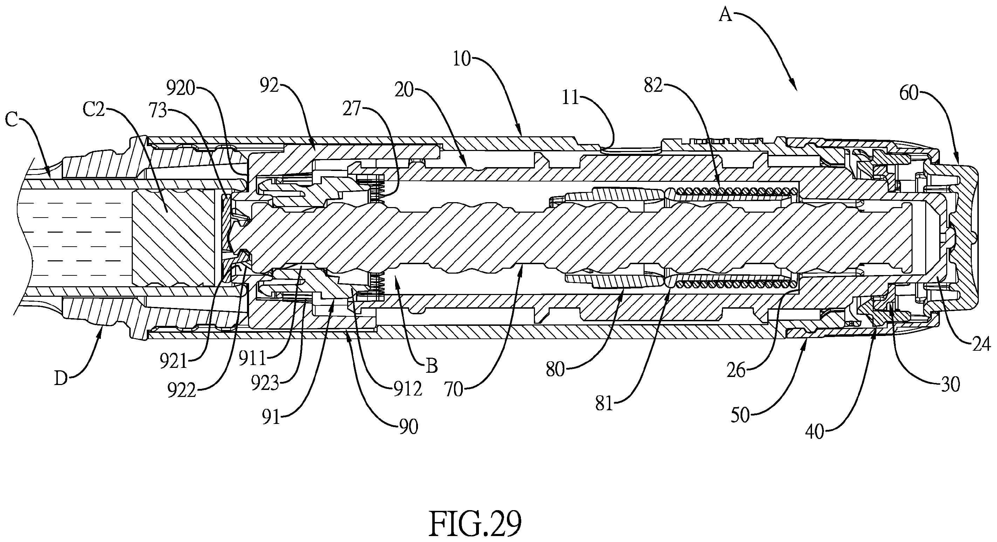

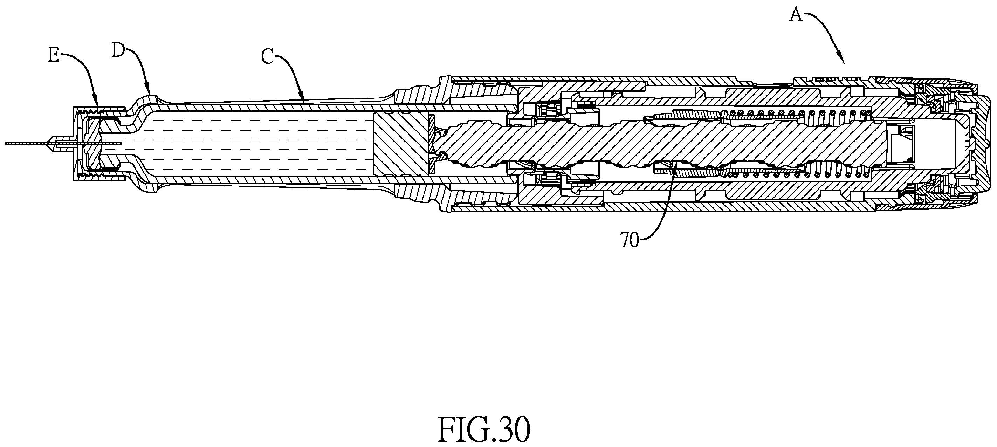

As shown in Figs. 26 and 28 to 31, before injection, the dose adjusting knob 50 is turned in the first direction (clockwise) first until a predetermined dose scale on the mounting sleeve 20 corresponds in position to the scale window 11 of the outer tube 10. Assuming the dose to be injected is 0.10mg, the dose adjusting knob 50 is turned to drive the dose scale "0.10- (mg)" of the mounting sleeve 20 to correspond in position to the scale window 11. Then, the needle E is pierced into an injection site of the human body and the pressing element 60 is pressed to perform the action of injection. The steps of setting the injection dose by rotation and performing the action of injection are as follows.

When turning the dose adjusting knob 50 to increase the injection dose, as the dose adjusting knob 50 drives the mounting sleeve 20 to rotate in the first direction (clockwise) to allow the predetermined dose scale to correspond in position to the scale window 11 of the outer tube 10, the dose adjusting knob 50 is turned in the first direction and the driving portion 52 of the dose adjusting knob 50 directly pushes the dividing columns 422 of the elastic transmission element 40 to drive the elastic transmission element 40 to rotate. Then the dividing columns 422 of the elastic transmission element 40 push the transmission ring 30 and the transmission ring 30 drives the mounting sleeve 20 to rotate in the same direction. With the two resilient ratchet arms 21 of the mounting sleeve 20 moving forward in the first direction on the one-way ratchet teeth 121 of the inner ratchet portion 12 of the outer tube 10, the engaging protrusion 210 of each of the two resilient ratchet arms 21 slides along the forward tooth face 1211 of one of the one-way ratchet teeth 121 and then re-engages in the next notch 120 by the resilience of the resilient ratchet arm 21, making the "beep" sound (the first sound).

During the process of turning the dose adjusting knob 50 to make incremental adjustment to the injection dose, the injection push rod 70 is limited by the orientation control element 92 that is secured in the outer tube 10 and the clutch control ring 91 that is limited in the orientation control element 92. On the other hand, the at least one axial guiding portion 802 of the driving ring 80 that is disposed in the mounting sleeve 20 protrudes in the at least one movable guiding groove 25 of the mounting sleeve 20. In addition to being axially movable in the mounting sleeve 20, the driving ring 80 also rotates along with the mounting tube 20. Thus, when the dose adjusting knob 50 is turned in the first direction to allow the dose scale "0.10-(mg)" to correspond in position to the scale window 11, the dose adjusting knob 50 also drives the mounting sleeve 20 to rotate in the first direction, the mounting sleeve 20 drives the driving ring 80 to move along the first spiral guiding groove 71 of the injection push rod 70 backward to rotationally move a distance to a position corresponding to the dose scale. Meanwhile, the driving ring 80 presses the resilient element 82 to store the resilient potential energy.

With the inner ratchet portion 12 mounted on the rear end of the outer tube 10 of the injection dose adjustment assembly A of the present invention, the two resilient ratchet arms 21 of the mounting sleeve 20 abut against two of the one-wary ratchet teeth 121 of the inner ratchet portion 12 of the outer tube 10. The dose adjusting knob 50 may be turned in the first direction (clockwise) or the second direction (counterclockwise) and drive the mounting sleeve 20 to rotate through the elastic transmission element 40 and the transmission ring 30, so as increase or decrease the injection dose and to adjust and control a displacement stroke of the injection push rod 70 of the injection assembly B. When the user over rotates the dose adjusting knob 50 in the first direction to increase the injection dose to be injected, the user may rotate the dose adjusting knob 50 reversely in the second direction to allow the predetermined dose scale to correspond in position to the scale window 11 of the outer tube 10 to decrease the injection dose to be injected.

In the process of decreasing the injection dose to be injected, the dose adjusting knob 50 is turned in the second direction (counterclockwise). With the triangular pushing protrusions 51 of the dose adjusting knob 50 corresponding in shape to the V-shaped bottoms of the coupling recesses 421 of the elastic transmission element 40, the mutual push between the triangular pushing protrusions 51 and the V-shaped bottoms of the coupling recesses 421 of the elastic transmission element 40 forms the axial force to push the elastic transmission element 40 to move forward when the dose adjusting knob 50 is turned in the second direction (counterclockwise). The axial force that forces the elastic transmission element 40 to move forward is transferred into the radial pushing force that is applied on the engaging protrusions 210 of the two resilient ratchet arms 21 of the mounting sleeve 20 toward the central axis. Thus, the engaging protrusions 210 are pushed away from the backward tooth faces 1212 of the one-way ratchet teeth 21 of the inner ratchet portion 12 of the outer tube 10 and are switched to the unlocked state that weakens the reverse block effect of the one-way ratchet teeth 121 applied on the engaging protrusions 210. Then the dose adjusting knob 50 continues to be turned in the second direction, and the driving portion 52 of the dose adjusting knob 50 pushes the dividing columns 422 of the elastic transmission element 40 to drive the elastic transmission element 40 and the transmission ring 30 to rotate. Further, the driving ring 30 drives the mounting sleeve 20 to rotate and to break through torque of the reverse block effect, which has been weakened due to the unlocked state, between the resilient ratchet arms 21 of the mounting sleeve 20 and the inner ratchet portion 12 of the outer tube 10. The two resilient ratchet arms 21 of the mounting sleeve 20 are restored by their own resilience to make the engaging protrusions 210 engage in the inner ratchet portion 12 of the outer tube 10, such that the "click" sound (the second sound) is emitted.

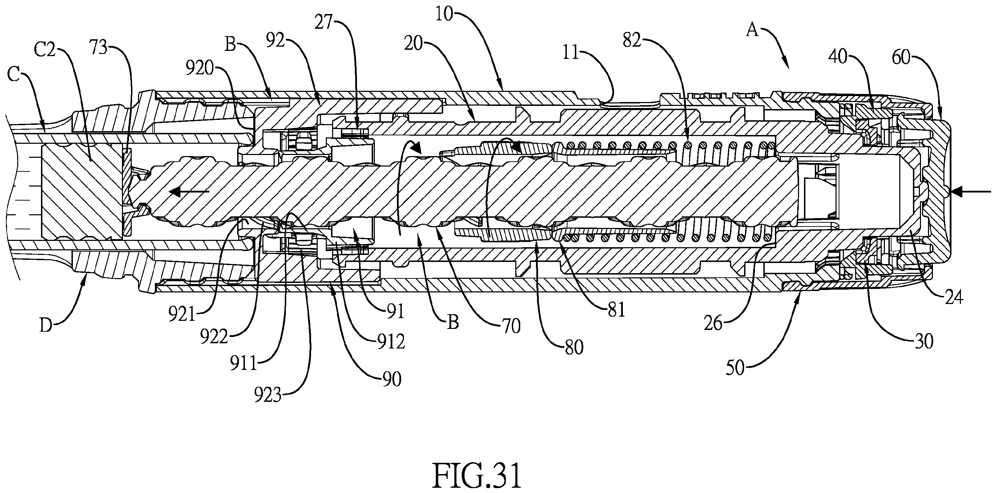

As shown in Figs. 1 and 31, when injecting the liquid medicament, a forward pressing force is applied to the pressing element 60 and lasts for a predetermined number of seconds. In the embodiment, the pressing force applied to the pressing element 60 lasts for about six seconds. By pushing the mounting sleeve 20 to move forward through the pressing element 60, the engaging toothed portion 23 on the rear end of the mounting sleeve 20 disengages from the inner annular engagement portion 32 of the transmission ring 30, such that the mounting sleeve 20 and the dose adjusting knob 50 do not move together. Then, the driving ring 80 is driven by the resilient potential energy stored in the resilient element 82 to move forward until the driving ring 80 contacts the clutch control ring 91. Meanwhile, with the first spiral portion 801 of the driving ring 80 coupled with the first spiral guiding groove 71 of the injection push rod 70 and with the help of the first spiral engaging portion 922 of the orientation control element 92 coupled with the first spiral guiding groove 71 of the injection push rod 70, the injection push rod 70 is driven by the resilient potential energy to spirally move forward. Then the piston abutting element 73 on the front end of the injection push rod 70 pushes the piston in the medicine vial C to move forward, so as to inject a predetermined amount of the liquid medicament in the medicine vial C into the human body through the needle E.

As described above, during the process of pushing the injection push rod 70 to spirally move forward, with the second spiral portion 911 of the clutch control ring 91 coupled with the second spiral guiding groove 72 of the injection push rod 70 and with the aligning notches 27 on the front end of the mounting sleeve 20 engaging and moving together with the aligning teeth 912 of the clutch control ring 91, the injection push rod 70 is driven by the resilient potential energy to spirally move forward and the clutch control ring 91 is driven to rotate in the second direction simultaneously. Meanwhile, the mounting sleeve 20 is also driven to rotate in the second direction until the dose scale "0.- (mg)" corresponds in position to the scale window 11 of the outer tube 10.

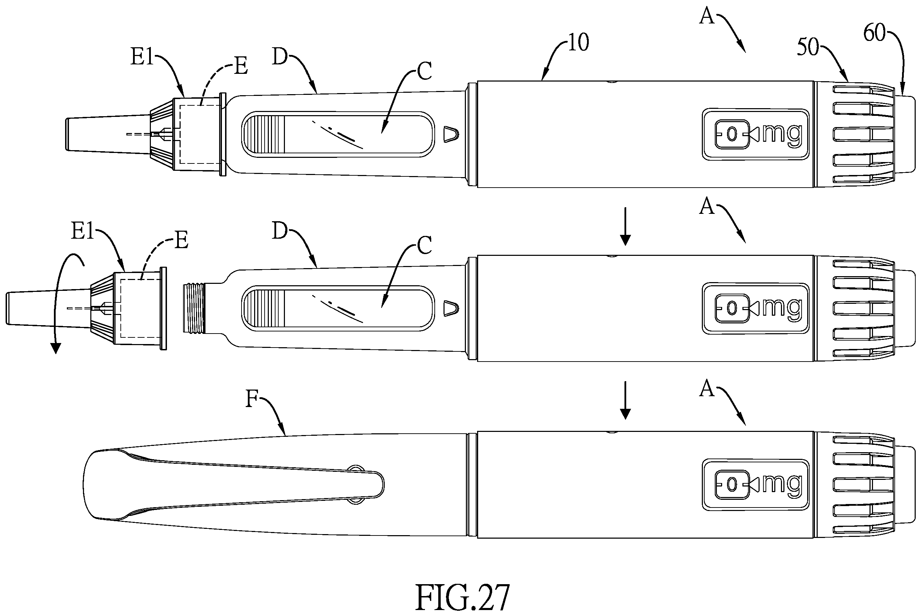

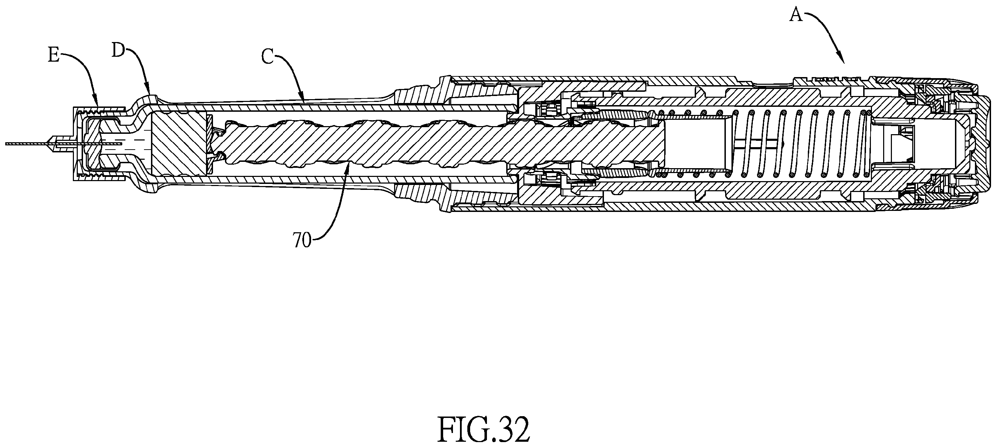

After completing the injection, as shown in Fig. 32, the driving ring 80 stays at a position of contacting a rear end of the clutch control ring 91. With a resilient force from the resilient element 82 pushing the mounting sleeve 20 to move backward, the aligning notches 27 of the front end of the mounting sleeve 20 disengage from the aligning teeth 912 of the clutch control ring 91, and the engaging toothed portion 23 on the rear end of the mounting sleeve 20 engages with the inner annular engagement portion 32 of the transmission ring 30 again. In this way, one injection process is completed. As shown in Fig. 27, after completion of the injection process, the needle protective sleeve E1 is mounted onto the needle E. The needle protective sleeve E1 is used as a safe auxiliary tool to remove the needle E, so as to safely remove the needle E from the front end of the vial mounting tube D. The needle E will not be reused. Then the protective cap F is mounted onto the vial mounting tube D and the injection mechanism is reserved for next use.

The injection syringe is used in the manner as described above until the liquid medicament in the medicine vial C is used up. In the injection mechanism, the orientation control element 92 secured in the outer tube 10 limits the clutch control ring 91 to be driven to rotate in the second direction only at the fixed position, and the second spiral portion 911 of the clutch control ring 91 is coupled with the second spiral guiding groove 72 of the injection push rod 70. Thus, when the dose adjusting knob 50 is turned, the injection push rod 70 is limited and is unable to rotate.

If the liquid medicament remaining in the medicine vial C is less than the predetermined injection dose, the dose adjusting knob 50 is unable to be further turned to allow the dose scale to correspond in position to the scale window 11. For the same reason, as shown in Fig. 31, when the liquid medicament in the medicine vial C is used up, the injection push rod 70 is also limited to prevent the dose adjusting knob 50 from being further rotated. As shown in Fig. 27, when the liquid medicament in the medicine vial C is used up, the needle protective sleeve E1 is mounted on the needle E. After the needle E is safely removed from the front end of the vial mounting tube D, the protective cap F is mounted onto the vial mounting tube D, so as to discard the injection syringe along with the used medicine vial C. The injection syringe is used within a limited number of times to ensure the safety of the injection syringe.

## Claims

1. An injection dose adjustment assembly (A), **characterized in** comprising:
an outer tube (10) having
an inner ratchet portion (12) disposed on a rear end of the outer tube (10);
multiple one-way ratchet teeth (121) formed on an inner sidewall of the inner ratchet portion (12) and extending radially, wherein a notch (120) is formed between each two of the one-way ratchet teeth (121) that are disposed next to each other;
a mounting sleeve (20) mounted in the outer tube (10), being axially movable, and being rotatable in a first direction and a second direction opposite to the first direction; and the mounting sleeve (20) having two resilient ratchet arms (21) being able to abut against two of the one-way ratchet teeth (121) of the inner ratchet portion (12) respectively;
an elastic transmission element (40) mounted on a rear end of the mounting sleeve (20) and being able to provide an axial elastic preload;
a dose adjusting knob (50) rotatably mounted on the rear end of the outer tube (10) and selectively engaging with or disengaging from the elastic transmission element (40); and
a pressing element (60) mounted in the dose adjusting knob (50) and being axially movable.

2. The injection dose adjustment assembly (A) as claimed in claim 1 further comprising: a transmission ring (30) mounted on the rear end of the mounting sleeve (20) and selectively engaging with or disengaging from the mounting sleeve (20) along an axial direction;
when the dose adjusting knob (50) is turned, the mounting sleeve (20) is driven to rotate through the elastic transmission element (40) and the transmission ring (30) and the pressing element (60) is able to trigger the mounting sleeve (20) to disengage from the transmission ring (30).

3. The injection dose adjustment assembly (A) as claimed in claim 1, wherein the dose adjusting knob (50) is capable of being turned in the first direction and the second direction;
when the dose adjusting knob (50) is turned in the second direction, the dose adjusting knob (50) pushes the elastic transmission element (40) to allow the elastic transmission element (40) to push the resilient ratchet arms (21) toward a central axis to form a state in which a block effect between the resilient ratchet arms (21) and the one-way ratchet teeth (121) is weakened.

4. The injection dose adjustment assembly (A) as claimed in claim 1, wherein in the inner ratchet portion (12) of the outer tube (10), each of the one-way ratchet teeth (121) has a forward tooth face (1211) facing toward the second direction and a backward tooth face (1212) facing toward the first direction; and the backward tooth face (1212) is steeper than the forward tooth face (1211).

5. The injection dose adjustment assembly (A) as claimed in claim 4, wherein each of the two resilient ratchet arms (21) has an engaging protrusion (210) formed on a distal end of the resilient ratchet arm (21); the engaging protrusions (210) of the two resilient ratchet arms (21) protrude in two of the notches (120) of the inner ratchet portion (12) of the outer tube (10) to occupy part of space in said two engaging protrusions (210); the engaging protrusion (210) of each of the resilient ratchet arms (21) has a first abutting surface (211) and a second abutting surface (212) inclined in different directions; the first abutting surface (211) of one of the resilient ratchet arms (21) abuts against the forward tooth face (1211), and the second abutting surface (212) of the other one of the resilient ratchet arms (21) abuts against the backward tooth face (1212).

6. The injection dose adjustment assembly (A) as claimed in claim 5, wherein
the dose adjusting knob (50) that is turned in the first direction is able to drive the engaging protrusions (210) of the two resilient ratchet arms (21) of the mounting sleeve (20) to slide along the forward tooth faces (1211) of the one-way ratchet teeth (121) to move forward from one said notch (120) to another and to emit a first sound; and
the dose adjusting knob (50) that is turned in the second direction is able to push the engaging protrusions (210) of the two resilient ratchet arms (21) away from the one-way ratchet teeth (21) through the elastic transmission element (40), such that the two resilient ratchet arms (21) are switched to the state in which the block effect is weakened, wherein when the engaging protrusions (210) of the two resilient ratchet arms (21) slide along the backward tooth faces (1212) of the one-way ratchet teeth (121) to move backward from one said notch (120) to another, a second sound, which is different from the first sound, is emitted.

7. The injection dose adjustment assembly (A) as claimed in claim 2, wherein
the elastic transmission element (40) has
an elastic portion (41) with compressive elasticity; and a front end of the elastic portion (41) abutting against the outer tube (10); and
an elasticity transmission annular portion (42) disposed on a rear end of the elastic portion (41) and being able to be coupled with the dose adjusting knob (50) and the transmission ring (30);
the dose adjusting knob (50) that is turned in the first direction is able to drive the mounting sleeve (20) to rotate in the first direction directly through the elastic transmission element (40) and the transmission ring (30); and
the dose adjusting knob (50) that is turned in the second direction is able to apply an axial force to the elasticity transmission annular portion (42), and the axial force is transferred into a radial pushing force that is applied on the two resilient ratchet arms (21) of the mounting sleeve (20) toward the central axis.

8. The injection dose adjustment assembly (A) as claimed in claim 7, wherein
each of the two resilient ratchet arms (21) has an engaging protrusion (210); the engaging protrusions (210) of the two resilient ratchet arms (21) protrude in two of the notches (120) of the inner ratchet portion (12) of the outer tube (10) to occupy part of space in said two engaging protrusions (210); the elasticity transmission annular portion (42) of the elastic transmission element (40) has multiple dividing columns (422) protruding backward; each of the dividing columns (422) has a connecting groove (423); the elasticity transmission annular portion (42) has multiple coupling recesses (421) and multiple annular backs (425); one of the coupling recesses (421) and one of the annular backs (425) are disposed between two of the dividing columns (422) that are disposed next to each other, and said annular back (425) is disposed on a side, which faces toward the second direction, of said coupling recess (421); the elasticity transmission annular portion (42) has a pushing portion (424);
an outer peripheral edge of the transmission ring (30) is provided with multiple connecting tabs (31); each of the connecting tabs (31) corresponds in position to and is assembled in the connecting grooves (423) of a corresponding one of the dividing columns (422);
multiple pushing protrusions (51) are disposed on an inner peripheral sidewall of the dose adjusting knob (50); each of the pushing protrusions (51) corresponds in position to and selectively engages with or disengages from a corresponding one of the coupling recesses (421); the dose adjusting knob (50) that is turned in the first direction is able to drive the elastic transmission element (40) and the transmission ring (30) to drive the mounting sleeve (20) to rotate in the first direction directly through the dividing columns (422);
in the dose adjusting knob (50) that is turned in the second direction, mutual push between the pushing protrusions (51) and the coupling recesses (421) forms the axial force forwardly to the elasticity transmission annular portion (42) and to transfer the axial force into the radial pushing force that is applied on the two resilient ratchet arms (21) of the mounting sleeve (20) toward the central axis through the pushing portion (424), such that the engaging protrusions (210) are pushed away from the one-way ratchet teeth (121) and the mounting sleeve (20) is driven to rotate in the second direction through the elastic transmission element (40) and the transmission ring (30).

9. The injection dose adjustment assembly (A) as claimed in claim 7, wherein the elastic portion (41) has
an annular portion (411) abutting against the outer tube (10); and
multiple elastic strips (412) rotationally symmetrically disposed between the elasticity transmission annular portion (42) and the annular portion (411); each of the elastic strips (412) extending obliquely and connected to the elasticity transmission annular portion (42) and the annular portion (411).

10. The injection dose adjustment assembly (A) as claimed in claim 8, wherein: each of the pushing protrusions (51) on the inner peripheral sidewall of the dose adjusting knob (50) is formed as a triangular block; and each of the coupling recesses (421) has a V-shaped bottom and corresponds in shape to the corresponding one of the pushing protrusions (51).

11. The injection dose adjustment assembly (A) as claimed in claim 10, wherein:
multiple driving portions (52) are provided on the inner peripheral sidewall of the dose adjusting knob (50); the driving portions (52) are positioned behind the pushing protrusions (51); a width of each of the driving portions (52) is smaller than a distance defined between two of the dividing columns (422) that are disposed next to each other; the dose adjusting knob (50) pushes the dividing columns (422) of the elastic transmission element (40) to drive the elastic transmission element (40) to rotate through the driving portions (52).

12. The injection dose adjustment assembly (A) as claimed in claim 1, wherein
the two resilient ratchet arms (21) are arranged in a non-rotational symmetry; each of the two resilient ratchet arms (21) has an engaging protrusion (210) formed on a distal end of the resilient ratchet arm (21); the engaging protrusions (210) of the two resilient ratchet arms (21) protrude in two of the notches (120) of the inner ratchet portion (12) of the outer tube (10) respectively and each of the engaging protrusions (210) occupies half of the space of a corresponding one of the notches (120); the engaging protrusion (210) of each of the resilient ratchet arms (21) has a first abutting surface (211) and a second abutting surface (212) inclined in different directions; and
a first connecting line (22A) is defined to pass through one of the resilient ratchet arms (21) and a central axis (100) of the outer tube (10), a second connecting line (22B) is defined to pass through the other one of the resilient ratchet arms (21) and the central axis (100) of the outer tube (10), and an included angle defined between the first connecting line (22A) and the second connecting line (22B) is half of a central angle defined by the notch (120) with the central axis (100) of the outer tube (10) as a center.

13. The injection dose adjustment assembly (A) as claimed in any one of claims 6, 8, 10 and 11, wherein the two resilient ratchet arms (21) are arranged in a non-rotational symmetry; the engaging protrusions (210) of the two resilient ratchet arms (21) protrude in two of the notches (120) of the inner ratchet portion (12) of the outer tube (10) respectively and each of the engaging protrusions (210) occupies half of the space of a corresponding one of the notches (120); a first connecting line (22A) is defined to pass through one of the resilient ratchet arms (21) and a central axis (100) of the outer tube (10), a second connecting line (22B) is defined to pass through the other one of the resilient ratchet arms (21) and the central axis (100) of the outer tube (10), and an included angle defined between the first connecting line (22A) and the second connecting line (22B) is half of a central angle defined by the notch (120) with the central axis (100) of the outer tube (10) as a center.

14. An injection mechanism capable of adjusting an injection dose, the injection mechanism configured to be connected to a medicine vial (C) containing a liquid medicament, and the injection mechanism **characterized in** comprising:
the injection dose adjustment assembly (A) as claimed in claim 2; and
an injection assembly (B) including
an injection push rod (70) mounted in the mounting sleeve (20) of the injection dose adjustment assembly (A) and being able to be driven to axially move forward to perform an action of injecting the liquid medicament in the medicine vial (C);
a driving ring (80) mounted in the mounting tube (20), being axially movable, and spirally connected with the injection push rod (70), wherein the mounting sleeve (20) is able to change a position of the driving ring (80) on the injection push rod (70);
a resilient element (82) mounted in the mounting sleeve (20) and disposed between the driving ring (80) and an inner wall of the rear end of the mounting sleeve (20) to provide a resilient force and to allow the mounting sleeve (20) to inter-connect with the transmission ring (30), wherein when the mounting sleeve (20) is rotated, a resilient potential energy is formed in the resilient element (82) that is driven by the driving ring (80); and
a clutch control unit (90) mounted inside a front end of the outer tube (10); when the dose adjusting knob (50) rotates the mounting sleeve (20), the clutch control unit (90) limits a position of the injection push rod (70); when the mounting sleeve (20) is triggered to be disconnected from the transmission ring (30), the clutch control unit (90) is able to be connected with the mounting sleeve (20); and when the injection push rod (70) completes the action of injection, the resilient element (82) drives the mounting sleeve (20) to disconnect from the clutch control unit (90) and be restored.

15. An injection syringe capable of adjusting injection dose, the injection syringe **characterized in** comprising:
the injection mechanism capable of adjusting the injection dose as claimed in claim 14; and
a vial mounting tube (D) sleeved on an outer side of the medicine vial (C) and detachably mounted on the front end of the outer tube (10).
